# EUROPEAN PATENT SPECIFICATION

(11) **EP 2 988 774 B1**
(45) Date of publication and mention of the grant of the patent: **05.06.2024**
(21) Application number: 14788364.9
(22) Date of filing: 22.04.2014
(51) Int. Cl.: A61K 39/00, A61K 39/38, C40B 30/04, A61K 35/17, G01N 33/50, A61P 43/00

(54) **TOLERIZING TREATMENTS FOR AUTOIMMUNE DISEASE**
TOLERANZ-INDUZIERENDE BEHANDLUNGEN FÜR AUTOIMMUNERKRANKUNGEN
TRAITEMENTS SENSIBILISANTS POUR MALADIE AUTO-IMMUNE

(30) Priority: 26.04.2013 US 201313871730
(43) Date of publication of application: 02.03.2016
(73) Proprietor: Enzo Biochem, Inc., Farmingdale, NY 11735 (US)
(72) Inventor: NUSSENBLATT, Robert, Bethesda, MD 20814 (US); LIU, Baoying, North Potomac, MD 20878 (US); WEI, Lai, Rockville, MD 20852 (US); RABBANI, Elazar, New York, NY 10003 (US); DONEGAN, James J., Amesbury, MA 01913 (US)
(74) Representative: Vossius & Partner Patentanwälte Rechtsanwälte mbB
(86) International application number: PCT/US2014/034948
(87) International publication number: WO 2014/176235

(56) References cited:
- EP-A1- 2 113 560
- WO-A1-2013/036914
- WO-A2-2009/090651
- US-A1- 2009 010 885
- US-A1- 2013 058 977
- US-B2- 7 947 464
- HOWARD L. WEINER ET AL: "Oral tolerance", IMMUNOLOGICAL REVIEWS., vol. 241, no. 1, 13 May 2011 (2011-05-13), pages 241-259, XP055302913, US ISSN: 0105-2896, DOI: 10.1111/j.1600-065X.2011.01017.x
- BHARATI MATTA ET AL: "Antigen-specific tolerance inhibits autoimmune uveitis in pre-sensitized animals by deletion and CD4+CD25+ T-regulatory cells", IMMUNOLOGY AND CELL BIOLOGY, vol. 88, no. 2, 3 November 2009 (2009-11-03), pages 187-196, XP055302955, AU ISSN: 0818-9641, DOI: 10.1038/icb.2009.83
- STEPHAN R. THURAU ET AL: "Oral tolerance with an HLA-peptide mimicking retinal autoantigen as a treatment of autoimmune uveitis", IMMUNOLOGY LETTERS., vol. 68, no. 2-3, 1 June 1999 (1999-06-01) , pages 205-212, XP055303248, NL ISSN: 0165-2478, DOI: 10.1016/S0165-2478(99)00071-1
- LU , L ET AL.: 'Major Peptide Autoepitopes For Nucleosome-Specific T Cells Of Human Lupus' JOURNAL OF CLINICAL INVESTIGATION. vol. 104, no. 3, August 1999, pages 345 - 355, XP055289116
- DESMET, MD ET AL.: 'Human S-Antigen Determinant Recognition In Uveitis.' INVESTIGATIVE OPHTHALMOLOGY & VISUAL SCIENCE . vol. 42, no. 13, December 2001, pages 3233 - 3238, XP055289118

## Description

### 1. STATEMENT REGARDING FEDERALLY SPONSORED RESEARCH

This work was made in part with Government support under Cooperative Research and Development Agreement (CRADA) Number 02491 and amendments thereto, executed between Enzo Therapeutics Incorporated and the National Eye Institute, National Institutes of Health.

### 2. BACKGROUND

The mammalian immune system has two contrasting functions that must co-exist for the health of the organism. On the one hand, the immune system recognizes foreign agents, *e.g.*, "non-self' agents such as bacteria or viruses that it attacks and destroys to restore health to an infected organism. On the other hand, the immune system recognizes the tissues of the organism and non-pathogenic "foreign" substances that are ingested (*e.g.*, food) so that the "self" is not attacked and the organism survives. In order for the regulation of these two functions to co-exist, the immune system must constantly correctly identify "self' and "non-self' to mount a proper response and to maintain a balance between action and selective inaction with respect to various challenges.

Autoimmune diseases result from an imbalance of the immune system, which becomes unable to distinguish "self' from "non-self" and mounts an inappropriate immune response to healthy tissues of the organism. The result of this imbalance is inflammation and tissue damage, which is often irreversible. Today, an autoimmune etiology is known or suspected to play a role in numerous seemingly unrelated diseases such as, uveitis, Crohn's disease, diabetes mellitus type I, lupus erythematosus, myasthenia gravis, psoriasis and rheumatoid arthritis. Increasing evidence suggests that immune mediated mechanisms also play an important role in the pathogenesis of age-related macular degeneration ("AMD"), the leading cause of blindness in the United States and the leading cause of blindness in people over 60 years of age. *See, e.g.,* Tarallo et al. (2012) Cell 149:847-859; Rosenbaum (2012) N Engl J Med. 367(8):768-770; Nussenblatt and Ferris (2007) AMD and the Immune System 144(4):618-626; Becerril et al. (2009) Cellular & Molecular Immunology 6(4):303-307. Accordingly, although AMD has traditionally been thought of as a disease confined to the eye, recent research suggests that it is a systemic immunological disease with local expression.

Current therapies for autoimmune diseases involve suppression of the immune system to mitigate the improper attack on "self' tissues. However, immune suppressive therapies tend to be non-selective, leading to inhibition of not only the aberrant autoimmune response, and but also of healthy responses to pathogens. Accordingly, immunosuppressive therapies can leave patients susceptible to infections, cancer and drug toxicity. Furthermore, suppression of the immune system only addresses one of the two functions of the immune system, which results in further unbalancing the system.

One approach for suppressing diseases that have an autoimmune component is induction of specific immune tolerance to soluble antigens by applying the soluble antigen to mucosal surfaces. *See e.g.,* Weiner et al. (2011) Immunol. Rev. 241(1):241-59. These tolerizing epitopes are administered to a patient in order to upregulate the functions of regulatory T-cells, which are T-cells with particular phenotypes that suppress responder T-cells, cells that are responsible for attacking agents that are recognized as "non-self'. While induction of regulatory T-cells by oral administration of a soluble antigen is considered to be a promising approach to treatment of autoimmune diseases, the ability to produce significant numbers of regulatory T-cells has been limited and requires identification of additional strategies (such as identification of a better antigen and/or co-administration of an enhancer of immune tolerance) to induce adequate numbers of functional regulatory cells. *See, e.g.,* Weiner at 249-50. Thurau et al., (1999) Immuno. Letters 68(2-3):205-212 describe the results of an open trial with nine patients by oral tolerance induction with the HLA-peptide B27PD in the treatment of therapy-refractive uveitis.

A more recent approach for suppressing autoimmune disease is to administer regulatory T-cells to the patient. *See, e.g.,* Marek-Trzonkowska et al. (2012) Diabetes Care 35:1817-20. In this study, regulatory autologous T-cells were expanded, but were not trained in the presence of an epitope. *Id.* at 1818. While this approach appears to have efficacy, it is not known whether it will provide long-term suppression of autoimmune disease. *Id.* at 1820. Furthermore, regulatory T-cell based therapies may be complicated by low numbers of regulatory T-cells in the body compared to other T-cells and their anergy, which means that they do not readily expand to provide enough cells for administration to a patient.

Accordingly, there is a need for improved methods and compositions that restore balance to the immune system of a patient suffering from an autoimmune disease by upregulating the regulatory function of the immune system.

### 3. SUMMARY

Provided is a pharmaceutical composition comprising the compound having SEQ ID NO: 1 for use in treating or preventing an autoimmune disease. In various aspects, the present disclosure is directed to a method of identifying a compound comprising an epitope that induces immune tolerance in a human patient suffering from an autoimmune disease comprising the step of identifying *in vitro* a compound from a library or collection of compounds that (a) elicits a response (RespH) from responder T-cells of a healthy individual; (b) elicits a response (RespP) from responder T-cells of the patient; (c) elicits a response (RegH) from regulatory T-cells of a healthy individual; and (d) elicits a response (RegP) from regulatory T-cells of the patient (RegP), wherein the compound that induces a response selected from a RespH/RespP<1, a RegH/RegP≥1, or a RespH/RespP<1 and a RegH/RegP≥1 is identified as the compound that induces immune tolerance. Accordingly, in certain aspects, a compound that induces immune tolerance is identified by a response in the presence of the compound of responder T-cells of a healthy individual that is lower than a response of responder T-cells of the patient. In other aspects, a compound that induces immune tolerance is identified by a response in the presence of the compound of regulatory T-cells of a healthy individual that is greater than or equal to a response of regulatory T-cells of the patient. In still other aspects, a compound that induces immune tolerance is identified by (i) a response in the presence of the compound of responder T-cells of a healthy individual that is lower than a response of responder T-cells of the patient; and (ii) a response in the presence of the compound of regulatory T-cells of a healthy individual that is greater than or equal to a response of regulatory T-cells of the patient.

In certain aspects, the disclosure relates to a method of identifying a compound comprising an epitope that induces immune tolerance in a human patient suffering from an autoimmune disease comprising the steps of (a) identifying *in vitro* a compound from a library or collection of compounds that (i) elicits a response (RespP₁) from responder T-cells of the patient; and (ii) elicits a response (RespH) from responder T-cells of a healthy individual wherein RespP₁/RespH >1 and (b) elicits a response (RespP₂) from responder T-cells of the patient in the presence of a responder T-cell antigen and regulatory T-cells, wherein RespP₂/RespP₁<1, wherein the compound that induces a RespP₁/RespH>1 and RespP₂/RespP₁< 1 is identified as the compound that induces an immune tolerance. Accordingly, in certain aspects, a compound that induces immune tolerance in a patient is identified by (i) a response in the presence of the compound of responder T-cells of the patient that is greater than a response from responder T-cells of the patient; and (ii) a response in the presence of the compound of responder T-cells of the patient in the presence of a responder T-cell antigen and regulatory T-cells that is lower than a response from responder T-cells of the patient in the absence of a responder T-cell antigen and regulatory T-cells.

In a more specific aspect, the disclosure relates to a method of identifying a compound comprising an epitope from a library or collection of compounds that induces immune tolerance in a human patient suffering from an autoimmune disease comprising the steps of (a) exposing human CD4⁺CD25⁺ cells to a compound from a library or collection of compounds; and (b) measuring the proliferation of said CD4⁺CD25⁺ cells in the presence of the compound (R₁); and (c) measuring the proliferation of said CD4⁺CD25⁺ cells in the absence of the compound (R₂), wherein the compound that induces R₁/R₂>1 is identified as the compound that induces immune tolerance. Accordingly, in some aspects, the a compound that induces immune tolerance in a patient is identified by a proliferation response of CD4⁺CD25⁺ cells in the presence of the compound that is greater than a proliferation response in the absence of the compound. In certain aspects, this assay is performed in the presence of an additional factor, such as IL-2.

In other aspects, the present disclosure relates to methods of identifying a compound comprising an epitope that induces immune tolerance in a patient suffering from an autoimmune disease using a mixed-cell assay. Thus, in some aspects, a compound comprising an epitope that induces immune tolerance in a patient suffering from an autoimmune disease is identified *in vitro* as the compound that elicits a response (RespP) from responder T-cells from the patient in the presence of regulatory T-cells from the patient that is greater than the response (RegP) elicited from the regulatory T-cells of the patient. Accordingly, in some aspects, an identified compound induces the response RespP/RegP>1.

In another aspect of a mixed-cell assay, a compound comprising an epitope that induces immune tolerance in a patient suffering from an autoimmune disease is identified *in vitro* as a compound that elicits a response (RegH) from regulatory T-cells from a healthy individual in the presence of responder T-cells from the patient that is greater than the response (RespP) elicited from the responder T-cells of the patient. Accordingly, in some aspects, an identified compound induces the response RegH>RespP.

In yet another aspect, a compound comprising an epitope that induces immune tolerance in a patient suffering from an autoimmune disease is identified *in vitro* as a compound that elicits a response (RespH) from responder T-cells from a healthy individual in the presence of regulatory T-cells from the patient that is greater than the response (RegP) elicited from the regulatory T-cells from the patient. Thus, in various aspects, an identified compound induces the response RespH>RegP.

In other aspects, a compound comprising an epitope that induces immune tolerance in a patient suffering from an autoimmune disease is identified *in vitro* as a compound that elicits a response (RespH) from responder T-cells from a healthy individual in the presence of regulatory T-cells from a healthy individual that is lower than the response (RegH) elicited from the regulatory T-cells from the healthy individual. In some aspects, an identified compound induces the response RespH<RegH.

In still other aspects, the present disclosure relates to a method of identifying a compound comprising an epitope that induces immune tolerance in a patient suffering from an autoimmune disease comprising the step of identifying *in vitro* a compound from a library or collection of compounds that (a) elicits a response (RespH) from responder T-cells of a healthy individual and (b) elicits a response (RespP) from responder T-cells of the patient, wherein the compound that induces a RespH/RespP<1 is identified as the compound that induces immune tolerance. Accordingly, in some aspects, the compound induces a response from responder T-cells of the patient that is greater than the response elicited from responder T-cells of a healthy individual.

The present disclosure further relates to uses of treating a patient suffering from an autoimmune disease. Thus, in some embodiments, the disclosure relates to a method of treating a human patient suffering from an autoimmune disease comprising administering to the patient an effective amount of regulatory T-cells. In certain specific aspects, the regulatory T-cells are trained *ex vivo* before administration to the patient in the presence of a compound comprising an epitope that induces immune tolerance, wherein the compound is identified from a library or collection of compounds, wherein the compound (a) elicits a response (RespH) from responder T-cells of a healthy individual; (b) elicits a response (RespP) from responder T-cells of the patient; (c) elicits a response (RegH) from regulatory T-cells of a healthy individual; and (d) elicits a response (RegP) from regulatory T-cells of the patient, and wherein the compound induces a response selected from a response of RespH/RespP<1, a response of RegH/RegP≥1 or a response of RespH/RespP<1 and RegH/RegP≥1. Accordingly, in certain aspects, the regulatory T-cells are trained in the presence of a compound that elicits a response from responder T-cells of a healthy individual that is lower than a response from responder T-cells of the patient. In other aspects, the regulatory T-cells are trained in the presence of a compound that elicits a response in the presence of the compound from regulatory T-cells of a healthy individual that is greater than or equal to a response of regulatory T-cells of the patient. In still other aspects, the regulatory T-cells are trained in the presence of a compound that (i) elicits a response in the presence of the compound from responder T-cells of a healthy individual that is lower than the response from responder T-cells of the patient; and (ii) elicits a response in the presence of the compound from regulatory T-cells of a healthy individual that is greater than or equal to a response from regulatory T-cells of the patient. In other specific aspects, the regulatory T-cells are expanded, but are not trained, before administration.

In other aspects, the disclosure relates to a combination therapy method of treating a patient suffering from an autoimmune disease comprising administering to the patient (a) an effective amount of regulatory T-cells; and (b) an effective amount of a compound comprising an epitope that induces immune tolerance. In certain aspects, the compound is identified from a library or collection of compounds, wherein the compound (i) elicits a response (RespH) from responder T-cells of a healthy individual and a response (RespP) from responder T-cells of the patient; (ii) elicits a response (RegH) from regulatory T-cells of a healthy individual and elicits a response (RegP) from regulatory T-cells of the patient, and wherein the compound induces a RespH/RespP<1 and a RegH/RegP>1. In certain aspects, the regulatory T-cells are trained *ex vivo* in the presence of a compound comprising an epitope that induces immune tolerance, wherein the compound is identified from a library or collection of compounds, wherein the compound (i) elicits a response (RespH) from a responder T-cell of a healthy individual and a response (RespP) from a responder T-cell of the patient; (ii) elicits a response (RegH) from a regulatory T-cell of a healthy individual and elicits a response (RegP) from a regulatory T-cell of the patient, and wherein the compound induces a RespH/RespP<1 and a RegH/RegP≥1. Accordingly, in certain aspects, the compound is identified as a compound that elicits a response from responder T-cells of a healthy individual that is lower than a response from responder T-cells of the patient. In other aspects, compound is identified as a compound that elicits a response in the presence of the compound from regulatory T-cells of a healthy individual that is greater than or equal to a response of regulatory T-cells of the patient. In still other aspects, the compound is identified as a compound that (i) elicits a response in the presence of the compound from responder T-cells of a healthy individual that is lower than the response from responder T-cells of the patient; and (ii) elicits a response in the presence of the compound from regulatory T-cells of a healthy individual that is greater than or equal to a response from regulatory T-cells of the patient. In other specific aspects, the regulatory T-cells are expanded, but are not trained, before administration.

In still other aspects, the present disclosure relates to a use in treating an autoimmune disease selected from age-related macular degeneration and uveitis in a patient wherein an effective amount of a compound comprising an epitope that induces immune tolerance comprising the step of administering a compound identified *in vitro* from a library or collection of compounds, wherein the compound (a) elicits a response (RespH) from responder T-cells of a healthy individual and (b) elicits a response (RespP) from responder T-cells of the patient, and wherein the compound induces a RespH/RespP<1 is to be administered. Accordingly, in certain aspects, the compound elicits a response from responder T-cells of a healthy individual that is lower than the response elicited from responder T-cells of the patient.

It should be noted that the indefinite articles "a" and "an" and the definite article "the" are used in the present application to mean one or more unless the context clearly dictates otherwise. Further, the term "or" is used in the present application to mean the disjunctive "or" or the conjunctive "and."

Any discussion of documents, acts, materials, devices, articles or the like that has been included in this specification is solely for the purpose of providing a context for the present disclosure. It is not to be taken as an admission that any or all of these matters form part of the prior art or were common general knowledge in the field relevant to the present disclosure as it existed anywhere before the priority date of this application.

The features and advantages of the disclosure will become further apparent from the following detailed description of embodiments thereof.

### 4. BRIEF DESCRIPTION OF THE FIGURES

FIG. 1 provides the results of lymphocyte proliferation studies in AMD patients and normal controls in response stimulation with human S-antigen peptides.
FIG. 2 provides the results of lymphocyte proliferation studies in AMD patients having small, intermediate and large drusen and normal controls in response to stimulation with human S-antigen peptide 23 ("P-23").

### 5. DETAILED DESCRIPTION

In certain aspects, methods are presented for identifying a compound comprising an epitope that induces immune tolerance in a patient suffering from an autoimmune disease. In certain aspects, the compounds are identified from a library or collection of compounds.

In other aspects, methods are provided for use in treating a patient suffering from an autoimmune disease. In various aspects, the patient is to be treated by administering an effective amount of regulatory T-cells that have been trained in the presence of a compound comprising an epitope that induces immune tolerance, wherein the compound is identified by a method described herein. In other aspects, the patient is to be treated by administering an effective amount of regulatory T-cells that are expanded, but have not been trained in the presence of a compound comprising an epitope that induces immune tolerance. In still other aspects, the patient is to be treated by administering an effective amount of regulatory T-cells that have not been trained *ex vivo.* In some aspects, the regulatory T-cells are trained *in vivo* upon administration of a compound comprising an epitope that induces immune tolerance, as identified by a method described herein. In particular aspects, the patient is to be treated with a mixture of regulatory T-cells from a healthy individual and regulatory T-cells from the patient.

In yet other aspects, methods are provided for use in treating a patient suffering from an autoimmune disease by administering an effective amount of a compound identified as described herein, and an effective amount of regulatory T-cells. In certain aspects, the regulatory T-cells are trained in the presence of a compound comprising an epitope that induces immune tolerance as identified by the methods described herein. In other aspects, the compound that is used to train the regulatory T-cells is different from the compound that is to be administered to the patient in this combination therapy. In certain aspects, the regulatory T-cells are expanded, but are not trained. In still other aspects, the regulatory T-cells are trained after administration of the T-cells to the patient by administration of a compound identified by a method described herein. In some aspects, the compound that is used to train the regulatory T-cells is the same compound that is to be administered to the patient.

As used herein, the term "patient" refers to humans and non-human animals. In some aspects, the patient suffers from an autoimmune disease due to one or more factors described herein. In certain aspects, the patient suffers from an autoimmune disease due to the presence of dysfunctional regulatory T-cells. As used herein, the term "dysfunctional" when referring to regulatory T-cells means that regulatory T-cell function in the patient is at least about 5%, at least about 10%, at least about 20%, at least about 30% or more lower than regulatory T-cell function in a healthy individual when comparing the same number of cells from the patient and the healthy individual. In other aspects, the patient suffers from an autoimmune disease due to the presence of lower numbers of regulatory T-cells as compared to numbers of regulatory T-cells in a healthy individual. In these aspects, the patient has at least about 5%, at least about 10%, at least about 20%, at least about 30% or more fewer regulatory T-cells than a healthy individual when comparing the numbers of T-cells in the same volume of blood. In still other aspects, the patient suffers from an autoimmune disease due to the presence of responder T-cells that are resistant to suppression by regulatory T-cells. In yet other aspects, the patient suffers from an autoimmune disease due to the presence of higher numbers of responder T-cells than in a healthy individual. In these aspects, the patient has at least about 5%, at least about 10%, at least about 20%, or at least about 30% or more responder T-cells than a healthy individual when comparing numbers of responder T-cells in the same volume of blood. In some aspects, the patient suffers from an autoimmune disease as a result of a combination of factors. *See e.g.,* Costantino et al. (2008) Eur. J. Immunol. 38(4):921-924; Baecher-Allan et al. (2004) Seminars in Immunol. 16:89-97.

The term "autoimmune disease" as used herein is any disease that arises from an inappropriate immune response of a patient's body against substances and tissues normally present in the body. In certain embodiments as defined in the claims, the autoimmune disease is selected from acute disseminated encephalomyelitis, Addison's disease, agammaglobulinemia, age-related macular degeneration, alopecia areata, amyotrophic lateral sclerosis, ankylosing spondylitis, antiphospholipid syndrome, antisynthetase syndrome, atopic allergy, atopic dermatitis, autoimmune aplastic anemia, autoimmune cardiomyopathy, autoimmune enteropathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune uticaria, autoimmune uveitis, Balo disease/Balo concentric sclerosis, Behçet's disease, Berger's disease, Bickerstaff's encephalitis, Blau syndrome, Bullous pemphigoid, cancer, Castleman's disease, celiac disease, Chagas disease, chronic inflammatory demyelinating polyneuropathy, chronic recurrent multifocal osteomyelitis, chronic obstructive pulmonary disease, Churg-Strauss syndrome, cicatricial pemphigoid, Cogan syndrome, cold agglutinin disease, complement component 2 deficiency, contact dermatitis, cranial arteritis, CREST syndrome, Crohn's disease, Cushing's syndrome, cutaneous leukocytoclastic angiitis, Dego's disease, Dercum's disease, dermatitis herpetiformis, dermatomyositis, diabetes mellitus type 1, diffuse cutaneous systemic sclerosis, Oressler's syndrome, drug-induced lupus, discoid lupus erythematosus, eczema, endometriosis, enthesitis-related arthritis, eosinophilic fasciitis, eosinophilic gastroenteritis, epidermolysis bullosa acquisita, erythema nodosum, erythroblastosis fetalis, essential mixed cryoglobulinemia, Evan's syndrome, fibrodysplasia ossificans progressive, fibrosing alveolitis, gastritis, gastrointestinal pemphigoid, glomerulonephritis, Goodpasture's syndrome, Graves' disease, Guillan-Barr& syndrome, Hashimoto's encephalopathy, Hashimoto's thyroiditis, Henoch-Schonlein purpura, gestational pemphigoid, hidradenitis suppurativa, Hughes-Stovin syndrome, hypogammaglobulinemia, idiopathic inflammatory demyelinating diseases, idiopathic pulmonary fibrosis, idiopathic thrombocytopenic purpura, IgA nephropathy, inclusion body myositis, chronic inflammatory demyelinating polyneuropathy, interstitial cystitis, juvenile idiopathic arthritis, Kawasaki's disease, Lambert-Eaton myasthenic syndrome, leukocytoclastic vasculitis, lichen planus, lichen sclerosus, linear IgA disease, lupus erythematosus, Majeed syndrome, Ménière's disease, microscopic polyangiitis, mixed connective tissue disease, morphea, Mucha-Habermann disease, multiple sclerosis, myasthenia gravis, myositis, narcolepsy, neuromyelitis optica, neuromyotonia, occular cicatricial pemphigoid, opsoclonus myoclonus syndrome, Ord's thyroiditis, palindromic rheumatism, pediatric autoimmune neuropsychiatric disorders associated with streptococcus, paraneoplastic cerebellar degeneration, paroxysmal nocturnal hemoglobinuria, Parry Romberg syndrome, Parsonage-Turner syndrome, Pars planitis, pemphigus vulgaris, pernicious anaemia, perivenous encephalomyelitis, POEMS syndrome, polyarteritis nodosa, polymyalgia rheumatic, polymyositis, primary biliary cirrhosis, primary sclerosing cholangitis, progressive inflammatory neuropathy,psoriasis, psoriatic arthritis, pyoderma gangrenosum, pure red cell aplasia, Rasmussen's encephalitis, Raynaud phenomenon, relapsing polychondritis, Reiter's syndrome, restless leg syndrome, retroperitoneal fibrosis, rheumatoid arthritis, rheumatic fever, sarcoidosis, schizophrenia, Schmidt syndrome, Schnitzler syndrome, scleritis, scleroderma, serum sickness, Sjögren's syndrome, spondyloarthropathy, stiff person syndrome, subacute bacterial endocarditis, Susac's syndrome, Sweet's syndrome, sympathetic ophthalmia, Takayasu's arteritis, temporal arteritis, thrombocytopenia, Tolosa-Hunt syndrome, transverse myelitis, ulcerative colitis, undifferentiated connective tissue disease, urticarial vasculitis, vasculitis, vitiligo and Wegener's granulomatosis.

In particular embodiments as defined in the claims, the autoimmune disease is selected from acute disseminated encephalomyelitis, age-related macular degeneration, alopecia areata, ankylosing spondylitis, antiphospholipid syndrome, autoimmune cardiomyopathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune uticaria, autoimmune uveitis, Behçet's disease, celiac disease, Chagas disease, chronic obstructive pulmonary disease, cold agglutinin disease, Crohn's disease, Dercum's disease, dermatomyositis, diabetes mellitus type 1, endometriosis, eosinophilic gastroenteritis, gastrointestinal pemphigoid, glomerulonephritis, Goodpasture's syndrome, Graves' disease, Guillan-Barré syndrome, Hashimoto's encephalopathy, Hasimoto's thyroiditis, hidradenitis suppurativa, idiopathic thrombocytopenic purpura, interstitial cystitis, Kawasaki's disease, lupus erythematosus, mixed connective tissues disease, morphea, multiple sclerosis, myasthenia gravis, narcolepsy, neuromyotonia, opsoclonus myoclonus syndrome, pediatric autoimmune neuropsychiatric disorders associated with streptococcus, paroxysmal nocturnal hemoglobinuria, pemphigus vulgaris, pernicious anaemia, polymyositis, primary biliary cirrhosis, progressive inflammatory neuropathy, psoriasis, psoriatic arthritis, Renaud phenomenon, relapsing polychondritis, restless leg syndrome, rheumatoid arthritis, rheumatic fever, sarcoidosis, schizophrenia, scleroderma, Sjögren's syndrome, stiff person syndrome, temporal arteritis, transverse myelitis, ulcerative colitis, undifferentiated connective tissue disease, vasculitis, vitiligo, and Wegener's granulomatosis.

In a particular embodiment as defined in the claims, the autoimmune disease is an autoimmune disease of the eye. In certain embodiments as defined in the claims, the autoimmune disease is selected from uveitis and age-related macular degeneration.

### 5.1. Methods of identifying a compound comprising an epitope that induces immune tolerance

As used herein, the term "compound comprising an epitope" includes a compound comprising a contiguous region of monomers that elicits immune tolerance in a patient suffering from an autoimmune disease. In certain aspects, the compound comprises an epitope (*i.e*., the epitope is a subset of contiguous monomers of the compound). In other aspects, the compound consists of the epitope (*i.e.*, the entire compound is the epitope). In various aspects, the epitope is a self-epitope of the patient. In other aspects, the epitope is a non-self epitope. In certain aspects, the epitope is organ specific. In other aspects, the epitope is not organ specific. In various aspects, the epitope is a human epitope. In other aspects, the epitope is a non-human mammalian epitope. In still other aspects, the epitope is a bacterial epitope or a viral epitope. In some aspects, the epitope is a mixture of epitopes from different organisms.

It will be understood by the skilled artisan that, in addition to the epitope, in various aspects, the compound includes one or more types of monomers, including but not limited to, naturally-occurring amino acids, non-naturally occurring amino acids, nucleotides, and the like. In certain aspects, the epitope consists of amino acids, which can be naturally occurring or non-naturally occurring. In particular aspects, the epitope consists of at least 3, such as at least 4, such as at least 5 or such as at least 6 or more amino acids. In certain aspects, the compound can be a single compound or an aggregate of compounds (*e.g*., cross-linked compounds). In various aspects, the compound can be unmodified or can be directly or indirectly (*i.e*., through a linking moiety) linked to another moiety, *e.g.,* a sugar, a fat, a label (*e.g.*, a fluorescent or radioactive label) or an additional therapeutic agent.

In various aspects, the compound comprising an epitope that induces immune tolerance in a patient is identified from a library or collection of compounds. In some aspects, the library is a library of biological epitopes. Accordingly, in certain aspects, the library is a library of organ specific epitopes. In these aspects, the epitopes are restricted to a particular organ of the body, *e.g*., the eye. Thus, in a particular aspect, the library of organ specific epitopes is a library of S-antigen epitopes. In other aspects, the library is a library of epitopes that are not organ specific, *e.g.,* that are found throughout the body. An example of this aspect is a library of HLA epitopes, such as a library of variant HLA epitopes (*e.g.,* a library of HLA-B27 epitopes). In some aspects, the library can be a library of epitopes from the patient (a library of self epitopes) or a library of epitopes that are not from the patient (a library of non-self epitopes).

In a particular aspect, the library is a library of peptides. In certain aspects, the library comprises synthetic peptides. In some aspects, peptides are synthesized with a given length and a predetermined overlapping sequence so that the library encompasses a particular protein. *See, e.g.,* Gershoni et al. (2007) BioDrugs 21 (3): 145-56. In other aspects, peptide libraries are created using mass spectrometry, such as by Solid Phase Epitope Recovery (SPHERE). *See* Lawendowski et al. (2002) J. Immunol. 169:2414-21. In certain aspects, a library for use in the methods described herein includes, but is not limited to a phage display library, a bacterial or yeast display library, an mRNA display library, a ribosomal display library, a polysomal display library and a peptide matrix. *See e.g.,* U.S. Patent Publication No. 2013/0004513 (Osterroth et al.).

In other aspects, the compound comprising an epitope that induces immune tolerance in a patient is identified from a collection of compounds. In these aspects, combinatorial epitope collections are utilized. Accordingly, in certain aspects, the collection comprises all permutations of a compound having 4 monomers. In certain aspects, the compound is a peptide and the collection comprises all permutations of a tetrameric peptide with all 20 amino acids at each position such that the collection includes 20⁴ peptide tetramers. In other aspects, the compound is a peptide and the collection comprises all permutations of a pentameric peptide with all 20 amino acids at each position such that the collection includes 20⁵ peptide pentamers.

In various aspects, the *in vitro* methods for identifying a compound comprising an epitope that induces immune tolerance in a patient suffering from an autoimmune disease comprise measuring responses of responder T-cells and regulatory T-cells from the patient and measuring responses of responder T-cells and regulatory T-cells from a healthy individual and comparing the various responses from the T-cells of the patient with responses from the T-cells of a healthy individual. As used herein, a "healthy individual" is an individual who does not suffer from an autoimmune disease.

As used herein, a "responder T-cell" or "T-resp" refers to T-cells that mount an immune response to antigens, such as antigens presented on antigen presenting cells. Specifically, T-resp cells referred to herein are cells that mount an immune response to antigens, and in this context, to self-antigens. T-resp cells can be polyclonal or antigen-specific. T-resp cells include T-cells with certain phenotypes, including, but not limited to, CD8⁺ cells, CD4⁺ T-cells, naive CD4⁺CD25⁻ T-cells, NK cells, cytotoxic T lymphocytes (CTL), and mature dendritic cells (DC).

As used herein, a "regulatory T-cell" or "T-reg" refers to T-cells that suppress an immune response of T-resp cells. In certain aspects, T-reg cells have an anergic phenotype, *i.e*., they do not proliferate in response to T-cell receptor stimulation. T-reg cells include T cells with particular phenotypes, including, but not limited to CD4⁺CD25⁺ T-cells, CD4⁺Foxp3⁺ T-cells, CD4⁺CD25⁺Foxp3⁺ T-cells, IL-10 producing CD4⁺ Tr1 cells, TGF-β producing Th3 cells, CD8⁺ NKT cells, CD4⁻CD8⁻ T-cells, γδ T-cells, thymic nT-reg cells, periphery induced i-Treg cells, tolerogenic dendritic cells (DC), CD4⁺CD127^{lo/-} T-cells, CD4⁺CD127^{lo/-}CD25⁺ T-cells, and the CD45RA⁺ subset of CD4⁺CD127^{lo/-}CD25⁺ T-cells. In various aspects, T-reg cells are negative for CD127 and positive for CD39. In other aspects, T-reg cells are induced from CD4⁺CD25⁻ cells by stimulation with irradiated allogenic stimulator PMBCs. In some aspects, the T-reg cells inhibit polyclonal T-resp cells. In other aspects, T-reg cells inhibit antigen-specific T-resp cells.

The skilled artisan will recognize that new phenotypes of T-reg cells and T-resp cells may be discovered. Accordingly, the present disclosure encompasses not only T-reg and T-resp cells as described above, but also any T-cell having the characteristics of T-reg cells or T-resp cells, whether identified herein or that are yet to be characterized.

Accordingly, in various aspects, the present disclosure relates to methods of identifying a compound comprising an epitope that induces immune tolerance in a human patient suffering from an autoimmune disease comprising the step of identifying *in vitro* a compound from a library or collection of compounds that (i) elicits a response (RespH) from a responder T-cell of a healthy individual, (ii) elicits a response (RespP) from a responder T-cell of the patient, (iii) elicits a response (RegH) from a regulatory T-cell of a healthy individual and (iv) elicits a response (RegP) from a regulatory T-cell of the patient, wherein the compound that induces a RespH/RespP<1, a RegH/RegP≥1 or a RespH/RespP<1 and a RegH/RegP≥1 is the compound that induces immune tolerance in the patient. In certain aspects, the compound induces a RespH/RespP≤1. In some aspects, the compound is identified by a RespP that is greater than the Resp H. In other aspects, the compound is identified by a RegH that is greater than the RegP. In still other aspects, the compound is identified by a RespP is greater than the Resp H and by a RegH that is greater than the RegP.

In another aspects, the disclosure relates to a method of identifying a compound comprising an epitope that induces immune tolerance in a patient suffering from an autoimmune disease comprising the steps of (a) identifying *in vitro* a compound from a library or collection of compounds that (i) elicits a response (RespP₁) from a responder T-cell of the patient; and (ii) elicits a response (RespH) from a responder T-cell of a healthy individual wherein RespP₁/RespH >1; and (b) elicits a response (RespP₂) from a responder T-cell of the patient in the presence of a responder T-cell antigen and a regulatory T-cell, wherein RespP₂/RespP₁< 1, and wherein the compound that induces a RespP₁/RespH >1 and RespP₂/RespP₁< 1 is identified as the compound that induces an immune tolerance.

In some aspects, the compound is identified by a RespP₁ that is greater than the RespH and by a RespP₁ that is greater than the RespP₂.

In yet another aspects, the disclosure relates to a method of identifying a compound comprising an epitope from a library or collection of compounds that induces immune tolerance in a human patient suffering from an autoimmune disease, comprising (i) exposing a human CD4⁺CD25⁺ cell to a compound, (ii) measuring the proliferation (Reg₁) of human CD4⁺CD25⁺ cells in the presence of the compound, and (iii) measuring the proliferation (Reg₂) of the human CD4⁺CD25⁺ cells in the absence of the compound, wherein the compound that induces Reg₁/Reg₂>1 is identified as the compound that induces immune tolerance in the patient. In a particular aspect , step (ii) is performed after the compound is removed. In certain aspects the compound that induces Reg₁/Reg₂≥1. In various aspects, the cell proliferation in the presence of the compound is greater than the cell proliferation in the absence of the compound.

In some aspects, the T-reg cells are induced, *e*.*g*., from naive cells, before step (i). In certain aspects, of this method, the CD4⁺CD25⁺ cells are isolated before being exposed to a compound. In some aspects, cells are isolated using commercially available isolation kits, such as magnetic bead isolation using antibodies that specifically bind to CD4 and/or CD25 and/or other cell surface markers. In certain aspects, kits using positive or a combination of negative and positive selection are used. In various aspects, the identification of specific T-cell phenotypes is carried out using flow cytometry. In a particular aspects, identification and/or separation is accomplished by FACs. In various aspects, the CD4⁺CD25⁺ cells are from a healthy individual.

In yet another aspects, the present disclosure relates to a method of identifying a compound comprising an epitope that induces immune tolerance in a patient suffering from an autoimmune disease, comprising identifying *in vitro* a compound from a library or collection of compounds that (i) elicits a response (RespH) from a T-resp of a healthy individual, and (ii) elicits a response (RespP) from a T-resp of the patient, wherein the compound that induces a RespH/RespP<1 is identified as the compound that induces immune tolerance in the patient. In some aspects, the compound induces a RespH/RespP≤1.

In certain aspects, the response of the responder T-cell of the patient in the presence of the compound is greater than the response of the responder T-cell of the healthy individual in the presence of the compound.

In various aspects, the present disclosure relates to a method of identifying a compound comprising an epitope that induces immune tolerance in a human patient suffering from an autoimmune disease using a mixed-cell assay. As used herein, the term "mixed-cell assay" refers to an assay that includes both (i) responsive T-cells and (ii) regulatory T-cells. Thus, in certain aspects, the method of identifying a compound comprises a step of identifying *in vitro* a compound that elicits a response (Presp) from a responder T-cell of a patient in the presence of regulatory T-cells of the patient that is greater than the response (Preg) elicited from the regulatory T-cells of the patient in the assay. In certain aspects, the Presp/Preg > 1. In other aspects, the method comprises a step of identifying *in vitro* a compound that elicits a response (Hreg) from regulatory T-cells of a healthy individual in the presence of responder T-cells of the patient that is greater than the response (Presp) elicited from the responder T-cells of the patient in the assay. In some aspects, Hreg/Presp>1. In still other aspects, the method comprises a step of identifying *in vitro* a compound that elicits a response (Hresp) from responder T-cells from a healthy individual in the presence of regulatory T-cells from the patient that is greater than the response (Preg) from the regulatory T-cells of the patient in the assay. In certain aspects, Hresp>Preg. In still other aspects, the method comprises a step of identifying *in vitro* a compound that elicits a response (Hresp) from responder T-cells from a healthy individual in the presence of regulatory T-cells from the healthy individual that is less than the response (Hreg) elicited from the regulatory T-cells of the healthy individual in the assay. In some aspects, Hresp<Hreg. It will be evident to the skilled artisan that more than one of the mixed-cell assays can be performed in order to identify a compound comprising an epitope that induces immune tolerance in a human patient suffering from an autoimmune disease.

As used herein, a "response" from at T-reg cell or a T-resp cell is an indication that a T-cell is upregulated. In various aspects, the response includes, but is not limited to, one or more of upregulation of cell-surface markers, such as activation markers, cytokine synthesis and/or secretion, and cell proliferation (expansion). A T-cell response can be measured by any method known in the art. In particular aspects, a T-cell response is measured by T-cell proliferation. In these aspects, T-cell proliferation is measured by cell counting, *e*.*g*., using flow cytometry, and in particular, fluorescence-activated cell sorting (FACs)) based on the T-cell markers. In other aspects, T-cell proliferation can be measured by [³H]-thymidine uptake. *See, e.g.,* Wallace et al. (2008) Cytometry A 73(11):1019-34. In certain aspects, T-cell proliferation can be measured using cell tracking dyes to label T-resp cells and monitor decreases in fluorescence associated with cell division. In some aspects in which a mixed-cell assay is used, T-reg cells and T-resp cells can be independently labeled with two readily distinguishable dyes in order to discriminate each T-cell population in co-cultures. *See* Brusko et al. (2007) Immunol. Investigations 36:607-628. *See, e.g.,* Venken et al. (2007) J. Immunol. Methods 322:1-11. In still other aspects, the activity of T-cells can be assayed by cytokine secretion, which can be detected, *e*.*g*., by an ELIspot assay. In still other aspects, activated T-cells can be assayed by detection of intracellular cytokine production by intracytoplasmic cytokine staining. Other assay formats for measuring T-cell responses will be known to the skilled artisan. *See, e.g.,* LiPira et al. (2010) J. Biomedicine and Biotechnol. 1-12. *See, e.g.,* Kruisbeek et al. Current Protocols in Immunology 3.12.1-3.12.20 (John Wiley & Sons, Inc., 2004).

In various aspects, the *in vitro* assays described herein are carried out in the absence of antigen presenting cells. In other aspects, the assays are performed in the presence of antigen presenting cells, such as murine antigen presenting cells or irradiated human PMBCs. In still other aspects, T-reg cells and/or T-resp cells are labeled, *e*.*g*., by radioisotopes or fluorescent dyes. In various aspects, the assays are performed in the presence of cytokines. Various types of *in vitro* T-cell assays for determining the activity of T-reg and T-resp cells will be known to the skilled artisan. *See, e.g.,* Collison and Vignali (2011) Methods Mol. Biol. 707:21-37.

The nature of T-reg cells in autoimmune diseases has been found to be variable. For example, Yeh et al., 2009 (Arch Opthamology 127; 407-413) found that there was a significant difference in numbers of T-reg cells between uveitis patients with active disease (4.3%) and uveitis patients with inactive disease (6.2%). Ursaciuc et al. 2010 (Romanian Arch Microbiol Immunol 69; 79-84) found a reduced presence of T-reg cells in systemic autoimmune diseases (SAID) compared to rheumatoid arthritis (RA) and controls and even concluded that T-reg percentage was the only cellular criterion of SAID evaluation. On the other hand, increased numbers of T-reg cells have also been found in autoimmune diseases such as juvenile arthritis (Cao et al., 2003 Eur J Immunol 33; 215-233). Lastly, there are reports that the defect lies not in the number of T-reg cells but in a disruption of their suppressive capability found in studies of multiple sclerosis (Viglietta et al., 2004 J Exp. Med. 199; 971-979), psoriasis (Sugiyama et al., 2005 J.Immunol. 174; 164-173) and myasthenia gravis (Baladina et al., 2003 AmN Y Acad Sci 998; 275-277). As such, certain aspects of the present disclosure measure the ability of a compound to be used in a suppressive assay with T-reg cells derived from diseased and healthy donors, where the number of total T-cells needed to provide a sufficient level of T-reg derived suppression of T-resp cells activity can be compared for a fixed level of inhibition. Thus, an antigen linked to a defect in either numbers or quality of T-reg cells in patients with autoimmune conditions will be recognized by these means since a ratio of the total number of T-cells from diseased and normal donors should be the same if the number and quality of T-reg cells are the same in both sources, whereas the number of T-cells used to achieve the fixed level will be greater to compensate for a loss of suppressive capability due to either a defect in the number or quality of T-reg cells in a patient sample. Accordingly, in various aspects, the measured response of T-cells is normalized. In some embodiments in which the patient has fewer T-reg cells than a healthy individual in the same volume of blood, the responder and/or regulatory T-cell response is normalized by the steps of (i) determining the total number of T-cells (all types) ("P1") from a healthy donor that provides an amount of T-reg cells that induces 50% suppression of the T-resp response; (ii) determining the total number of T-cells (all types) ("P2") from the donor suffering from an autoimmune disease that provides an amount of T-reg cells that induces suppression of 50% of the T-resp response; and (iii) calculating P1/P2 to determine the amount of T-reg cells that are lacking in the donor suffering from an autoimmune disease. A ratio of P1/P2 that is greater than 1 is an indication that the compound may have therapeutic value as a tolerogenic agent or an agent for inducing or expanding T-reg cells that recognize the compound.

Thus, in some aspects, the disclosure relates to a method of identifying a compound comprising an epitope that induces immune tolerance in a human patient suffering from an autoimmune disease comprising the steps of (a) identifying *in vitro* a compound from a library or collection of compounds that (i) elicits a response (RespH) from responder T-cells of a healthy individual; and (ii) elicits a response (RespP) from responder T-cells of the patient; (b) determining the total number of T-cells (P1) from the healthy individual that provides an amount of T-reg cells that induces 50% suppression of T-resp activity in the presence of said compound; and (c) determining the total number of T-cells (P2) from the patient that provides an amount of T-reg cells that induces 50% suppression of said T-resp activity in the presence of said compound, wherein the compound that induces a RespH/RespP<1, a P1/P2>1 or RespH/RespP<1 and a P1/P2>1 is identified as the compound that induces immune tolerance in the patient.

In other aspects, the screening methods identify an epitope from a library of biological epitopes for treating age-related macular degeneration. In various aspects, the compound is a peptide. In a particular embodiment as defined in the claims, the peptide has the sequence N-GEPIPVTVDVTNNTEKTVKK-C, the P-23 fragment of S-antigen ("P-23"). Accordingly, the present disclosure also provides uses in treating a patient suffering from an autoimmune disease of the eye. In certain embodiments as defined in the claims, the autoimmune disease is selected from uveitis and age-related macular degeneration. "Age-related macular degeneration" or "AMD" as used herein encompasses all forms of the disease, including dry AMD and wet AMD, and disease at any stage, such as, for example, dry AMD in patients with small, intermediate or large drusen volumes.

In some aspects, an assay described herein is performed in the presence of one or more additional agents. In certain aspects, an assay described herein is performed in the presence of an immune tolerance enhancer. As used herein, an "enhancer" is any compound or mixture of compounds that potentiates the immune suppressive response of T-reg cells. In certain aspects, the enhancer is required for T-reg cell expansion. In some aspects, the enhancer is used in the *in vitro* methods described herein. In other aspects, the enhancer is used in *in vivo* methods described herein. In still other aspects, the enhancer is used in both *in vitro* assays and *in vivo* methods. In some aspects, the enhancer is high molecular weight hyaluronic acid. As used herein, the term "high molecular weight hyaluronic acid" refers to hyaluronic acid having a molecular weight of at least about 1 × 10⁶ Da, such as of at least about 2 × 10⁶ Da, at least about 3 × 10⁶ Da, at least about 4 × 10⁶ Da, or more. *See e.g.,* Bollyky et al. (2007) J. Immunol. 179:744-747. Other enhancers include IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof. *See, e.g.,* Viney et al. (1998) J. Immunol. 160(12):5815-25; Horwitz et al. (2004) Seminars in Immunol. 16:135-143; Daniel et al. (2007) J. Immunol. 178(2): 458-68; Weiner et al. (2011) Immunol Rev. 241(1):241-259; Ma et al. (2011) Int. Immunopharmacol. 11(5):618-29; Adriouch et al. (2011) Front. Microbiol. 2:199; Dons et al. (2012) Human Immunol. 73:328-334 . In certain aspects, the enhancer is a sphingosine kinase 1 inhibitor as disclosed in U.S. Patent No. 8,872,888. It will be understood by the skilled artisan that newly discovered enhancers are contemplated for use in the instant disclosure .

In some aspects, the compound identified by a method described above is used as a reference sequence to search a library for additional compounds, which have homology to the reference sequence. In certain aspects, the reference sequence is the entire protein target sequence. In various aspects, the reference sequence and identified compounds are compared using a comparison window, a contiguous specific segment of the polypeptide sequence, which can have gaps compared to the reference sequence, for optimal alignment of peptides. In certain aspects, the comparison sequence is at least about 10 amino acids, at least about 15 amino acids, at least about 20 amino acids, or at least about 25 or more amino acids. Tools for aligning sequences for comparison are well known in the art and include, but are not limited to, CLUSTAL in the PC/Gene program (available from Intelligenetics, Mountain View, Calif.); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, Calif., USA). In certain aspects, compounds are chosen that at least about 30%, such as at least about 35%, at least about 40%, at least about 45%, at least about 50%, at least about 55%, at least about 60%, at least about 65%, at least about 70%, at least about 75%, at least about 80%, at least about 85%, or at least about 90% or more homology or identity to the reference sequence. The identified compound can then be assayed by a method described herein.

It will be understood by the skilled artisan that a compound identified by the methods described herein may be optimized to improve efficacy by altering or augmenting certain properties. Accordingly, in some aspects, a compound may be optimized, *e*.*g*., to improve solubility, absorption and/or stability of the compound, to prolong its half-life in the body, or to target a specific organ. In some aspects, optimization may include altering the molecular weight, length and chemical make-up of the compound. In one aspect, the compound is a peptide. In these aspects, a peptide can be optimized by, *e.g*., adding or removing amino acids, introducing conservative or non-conservative amino acid substitutions at various positions, incorporating non-natural amino acids, and crosslinking to other peptides or non-peptide therapeutic agents.

The skilled artisan will appreciate that optimization of a compound identified by the methods set forth herein may be an iterative process, comprising alteration of the compound followed by retesting of the altered compound in an *in vitro* assay described herein.

### 5.2. Uses in treatment

The phrases "treatment of," "treating", and the like include the amelioration or cessation of a condition or a symptom thereof. In one aspect, treating includes inhibiting, for example, decreasing the overall frequency of episodes of a condition or a symptom thereof. In various aspects, the condition is an autoimmune disease.

The phrases "prevention of," "preventing", and the like include the avoidance of the onset of a condition or a symptom thereof.

In accordance with the disclosure , in some aspects, the compounds described herein are to be administered to a patient in need of treatment or prevention of an autoimmune disease. In some aspects, the compounds are to be administered to a patient in need of treatment or prevention of age-related macular degeneration. As used herein, the term "patient" includes, but is not limited to, a human or a non-human animal.

In certain aspects, a patient suffering from an autoimmune disease is to be treated by administering an effective amount of regulatory T-cells trained in the presence of a compound comprising an epitope that induces immune tolerance, wherein the compound is identified as the compound that (a) elicits a response (RespP) from a responder T-cell of the patient; (b) elicits a response (RespH) from a responder T-cell of the patient; (c) elicits a response (RegH) from a regulatory T-cell of a healthy individual; and (d) elicits a response (RegP) from a regulatory T-cell of the patient, wherein the compound induces a RespH/RespP<1, a RegH/RegP≥1 or a RespH/RespP<1 and a RegH/RegP≥1. As used herein, a cell is "trained" when it is exposed to a compound identified by the methods described herein. In various aspects, the phenotype of the cell is altered upon exposure to a compound identified by the methods described herein. In other aspects, the regulatory T-cells are not trained before administration. In various aspects, the regulatory T-cells are expanded but not trained. In still other aspects, the regulatory T-cells are trained *in vivo* by administration of a compound identified by the methods described herein before, concurrently with or subsequent to administration of the regulatory T-cells.

In other aspects, the patient is to be treated by (a) administering an effective amount of regulatory T-cells and (b) administering a compound that (i) elicits a response (RespP) from a responder T-cell of the patient; (ii) elicits a response (RespH) from a responder T-cell of the patient; (iii) elicits a response (RegH) from a regulatory T-cell of a healthy individual; and (iv) elicits a response (RegP) from a regulatory T-cell of the patient, wherein the compound induces a RespH/RespP<1 and a RegH/RegP≥1. In certain aspects, the T-cells to be administered in step (a) are trained and the compound used to train them is identical to the compound administered in step (b). In other aspects, the compound used to train the T-reg cells to be administered in step (a) is different from the compound to be administered in step (b). In one particular aspect, the T-reg cells are not trained. In some aspects, the T-reg cells are expanded but not trained. Accordingly, in some aspects, the compound is to be administered after administration of the T-reg cells in order to train and/or maintain the T-reg cell population in the patient.

### 5.3. Compositions and administration of compounds comprising an epitope that induces immune tolerance

when to be administered to a patient, a compound identified by the methods described herein can be administered as a component of a composition that comprises a pharmaceutically acceptable carrier or excipient. Compositions comprising the compound can be administered by absorption through mucocutaneous linings (*e.g.*, oral, rectal, and intestinal mucosa, etc.). Administration can be systemic or local. Methods of administration include, but are not limited to, oral, sublingual, intravaginal, rectal, by inhalation and parenterally.

In particular aspects, the compound is to be administered with an enhancer. As used herein, an "enhancer" is any compound or mixture of compounds that potentiates the immune suppressive response of T-reg cells. In some aspects, the enhancer is high molecular weight hyaluronic acid. As used herein, the term "high molecular weight hyaluronic acid" refers to hyaluronic acid having a molecular weight of at least about 1 × 10⁶ Da, such as of at least about 2 × 10⁶ Da, at least about 3 × 10⁶ Da, at least about 4 × 10⁶ Da, or more. *See e.g.,* Bollyky et al. (2007) J. Immunol. 179:744-747. Other enhancers include
IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof. *See, e.g.,* Viney et al. (1998) J. Immunol. 160(12):5815-25; Horwitz et al. (2004) Seminars in Immunol. 16:135-143; Daniel et al. (2007) J. Immnol. 178(2): 458-68; Weiner et al. (2011) Immunol Rev. 241(1):241-259; Ma et al. (2011) Int. Immunopharmacol. 11(5):618-29; Adriouch et al. (2011) Front. Microbiol. 2:199; Dons et al. (2012) Human Immunol. 73:328-334 . In certain aspects, the enhancer is a sphingosine kinase 1 inhibitor as disclosed in U.S. Patent No. 8,872,888 .

The compositions described herein can optionally comprise a suitable amount of a pharmaceutically acceptable excipient so as to provide the form for proper administration to the patient. Examples of pharmaceutical excipients include a diluent, suspending agent, solubilizer, binder, disintegrant, preservative, coloring agent, lubricant, and the like. The pharmaceutical excipient can be a liquid, such as water or an oil, including those of petroleum, animal, vegetable, or synthetic origin, such as peanut oil, soybean oil, mineral oil, sesame oil, and the like. The pharmaceutical excipient can be saline, gum acacia, gelatin, starch paste, talc, keratin, colloidal silica, urea, and the like. In addition, auxiliary, stabilizing, thickening, lubricating, and coloring agents can be used. In one aspect, the pharmaceutically acceptable excipient is sterile when to be administered to a patient. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. The disclosed compositions, if desired, can also contain minor amounts of wetting or emulsifying agents, or pH buffering agents. Specific examples of pharmaceutically acceptable carriers and excipients that can be used to formulate oral dosage forms are described in the Handbook of Pharmaceutical Excipients, American Pharmaceutical Association (1986).

The disclosed compositions can take the form of solutions, suspensions, emulsions, tablets, pills, pellets, capsules, capsules containing liquids, powders, sustained-release formulations, suppositories, emulsions, aerosols, sprays, suspensions, or any other form suitable for use. Examples of suitable pharmaceutical excipients are described in Remington's Pharmaceutical Sciences 1447-1676 (Alfonso R. Gennaro ed., 19th ed. 1995).

In one aspect, the compounds are formulated in accordance with routine procedures as a composition adapted for oral administration. A compound to be orally delivered can be in the form of tablets, capsules, gelcaps, caplets, lozenges, aqueous or oily solutions, suspensions, granules, powders, emulsions, syrups, or elixirs, for example. When a compound is incorporated into oral tablets, such tablets can be compressed tablets, tablet triturates (e.g., powdered or crushed tablets), enteric-coated tablets, sugar-coated tablets, film-coated tablets, multiply compressed tablets or multiply layered tablets. Techniques and compositions for making solid oral dosage forms are described in Pharmaceutical Dosage Forms: Tablets (Lieberman, Lachman and Schwartz, eds., 2nd ed.) published by Marcel Dekker, Inc. Techniques and compositions for making tablets (compressed and molded), capsules (hard and soft gelatin) and pills are also described in Remington's Pharmaceutical Sciences 1553-1593 (Arthur Osol, ed., 16th ed., Mack Publishing, Easton, PA 1980).

Liquid oral dosage forms include aqueous and nonaqueous solutions, emulsions, suspensions, and solutions and/or suspensions reconstituted from non-effervescent granules, optionally containing one or more suitable solvents, preservatives, emulsifying agents, suspending agents, diluents, sweeteners, coloring agents, flavoring agents, and the like. Techniques and composition for making liquid oral dosage forms are described in Pharmaceutical Dosage Forms: Disperse Systems, (Lieberman, Rieger and Banker, eds.) published by Marcel Dekker, Inc.

A to be orally administered compound can contain one or more agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving compound are also suitable for orally administered compositions. A time-delay material such as glycerol monostearate or glycerol stearate can also be used. Oral compositions can include standard excipients such as mannitol, lactose, starch, magnesium stearate, sodium saccharin, cellulose, and magnesium carbonate.

Alternatively, when a compound is to be inhaled, it can be formulated into a dry aerosol or can be formulated into an aqueous or partially aqueous solution.

In various aspects, a compound is to be administered parenterally, *e.g*., intravenously or by injection. When a compound is to be injected parenterally, it can be in the form of, *e.g.*, an isotonic sterile solution.

The amount of compound that is effective for use in the treatment or prevention of a condition can be determined by standard clinical techniques. In addition, *in vitro* and/or *in vivo* assays can optionally be employed to help identify optimal dosage ranges. The precise dose to be employed will also depend on, *e.g.,* the route of administration and the seriousness of the condition, and can be decided according to the judgment of a practitioner and/or each patient's circumstances. In other examples thereof, variations will necessarily occur depending upon the weight and physical condition (*e.g*., hepatic and renal function) of the patient to be treated, the affliction to be treated, the severity of the symptoms, the frequency of the dosage interval, the presence of any deleterious side-effects, and the particular compound utilized, among other things.

Administration can be as a single dose or as a divided dose. In one aspect, an effective dosage is to be administered once per month until the condition is abated. In another aspect, the effective dosage is to be administered once per week, or twice per week or three times per week until the condition is abated. In another aspect, an effective dosage amount is to be administered about every 24 h until the condition is abated. In another aspect, an effective dosage amount is to be administered about every 12 h until the condition is abated. In another aspect, an effective dosage amount is to be administered about every 8 h until the condition is abated. In another aspect, an effective dosage amount is to be administered about every 6 h until the condition is abated. In another aspect, an effective dosage amount is to be administered about every 4 h until the condition is abated. The effective dosage amounts described herein refer to total amounts to be administered; that is, if more than one compound is to be administered, the effective dosage amounts correspond to the total amount to be administered.

In various aspects, the compound can be administered together with a second therapeutically active agent. In some aspects, the additional agent is a dietary supplement such as a vitamin, a mineral, or an ω-3 fatty acid. In other aspects, the second therapeutically active agent is an anti-inflammatory agent, *e.g.,* a corticosteroid.

In one aspect, a compound is to be administered concurrently with a second therapeutic agent as a single composition comprising an effective amount of the compound and an effective amount of the second therapeutic agent. Alternatively, a composition comprising an effective amount of a compound and a second composition comprising an effective amount of the second therapeutic agent are concurrently to be administered. In another aspect, an effective amount of a compound is to be administered prior or subsequent to administration of an effective amount of the second therapeutic agent. In this aspect, the compound is to be administered while the second therapeutic agent exerts its therapeutic effect, or the second therapeutic agent is to be administered while the compound exerts its therapeutic effect for use in treating or preventing a Condition.

An effective amount of the second therapeutic agent will be known to the art depending on the agent. However, it is well within the skilled artisan's purview to determine the second therapeutic agent's optimal effective-amount range. In some aspects of the disclosure, where a second therapeutic agent is to be administered to a patient for treatment of a condition, the minimal effective amount of the compound will be less than its minimal effective amount would be where the second therapeutic agent is not to be administered. In this aspect, the compound and the second therapeutic agent can act synergistically for use in treating or preventing a condition.

A composition of the disclosure is prepared by a method comprising admixing a compound or a pharmaceutically acceptable derivative thereof with a pharmaceutically acceptable carrier or excipient. Admixing can be accomplished using methods known for admixing compounds, *e.g*., a peptide, and a pharmaceutically acceptable carrier or excipient. In one embodiment, the compound is present in the composition in an effective amount.

### 5.4. Cell-based therapy

In certain aspects, a patient suffering from an autoimmune disease is to be treated by administering T-reg cells. In some embodiments, the T-reg cells are autologous T-reg cells isolated from the patient. In other aspects, the T-reg cells are heterologous T-reg cells isolated from a healthy individual. In these aspects, the heterologous T-reg cells are compatible with the patient. As used herein, a heterologous T-cell is "compatible" with the patient if it is isolated from a partially HLA-matched individual. In some aspects, the T-reg cells are cryopreserved cells. In particular aspects, the T-reg cells are from a cell bank. In other particular aspects, the T-reg cells are derived from undifferentiated umbilical cord stem cells.

In various aspects, the T-reg cells are trained before administration to a patient. In other aspects, the T-reg cells are not trained before administration to a patient. In some aspects, the T-reg cells are trained *in vitro* in the presence of a compound identified by the methods described herein. In other aspects, the T-reg cells are trained *in vivo* after administration, for example, by administering a compound identified by the methods described herein before, concurrently with or subsequent to administration of the T-reg cells. In various aspects, the T-reg cells are expanded but are not trained. In still other aspects, the T-reg cells that are not expanded are infused into the blood of a patient and are expanded *in vivo.*

In some aspects, precursor cells are induced to become T-reg cells. In some aspects, the precursor cells are induced in the presence of a compound identified by the methods described herein. In other aspects, the precursor cells are induced to become T-reg cells, and the induced T-reg cells are subsequently trained with a compound identified by the methods described herein. In particular aspects, the induced T-reg cells are expanded *in vitro* in the presence of a compound identified by the methods described herein. In various aspects, the induced T-reg cells are expanded *in vivo* by administering induced T-cells to the patient and administering an effective amount of a compound as identified by a method described herein before, concurrently with or subsequent to administering the T-cells. In some aspects, the precursor cells are autologous cells of the patient. In other aspects, the precursor cells are heterologous cells that are compatible with the patient. In particular aspects, the heterologous precursor cells are from a healthy individual. In various aspects, the induced T-reg cells are antigen specific. In other aspects, the induced T-reg cells are polyclonal.

In various aspects, T-reg cells or precursor cells are isolated from the peripheral blood. In some aspects, T-reg cells or precursor cells are isolated from fresh peripheral blood. In other aspects, T-reg cells or precursor cells are isolated from cryopreserved peripheral blood. In still other aspects, T-reg cells or precursor cells are isolated from umbilical cord stem cells.

Regulatory T-cell populations useful in the cell therapies described herein will be evident to the skilled artisan. Such cells include, but are not limited to, natural CD4⁺CD25⁺ thymus-derived T-cells, natural CD4⁺CD25⁺Foxp3⁺ cells, CD4⁺CD25⁺ T-cells induced *ex vivo* by stimulation of CD4⁺ cells induced with alloantigens in the presence of TGF-β, CD4⁺, Tr1 cells induced *ex vivo* with mitogens and IL-10 or immature dendritic cells, CD4⁺ Th3/Tr2 cells induced *ex vivo* with mitogens or superantigens in the presence of IL-2 and TGF-β, CD8⁺ Tr1 or Tr2 cells induced *ex vivo* with mitogens in the presence of IL-10 or TGF-β with plasmacytoid dendritic cells, CD4⁺ T-cells stimulated with anti-CD3 and complement regulator anti-CD46, CD4⁺CD25⁻ cells induced by specific antigens (*e.g*., HLA class II tetramers) or TGF- β, TGF-β converted CD4⁺CD25⁺ T-cells, CD4⁺CD25⁺CD127^{low} T-cells, CD4⁺CD127^{lo/-} T-cells, CD4⁺CD127^{lo/-}CD25⁺ T-cells, and the CD54RA⁺ subset of CD4⁺CD127^{lo/-}CD25⁺ T-cells. Other T-cells that can be used in the methods described herein will be known to the skilled artisan, and can be found, for example in Horwitz et al. (2004) Seminars in Immunology 16:135-143. *See also* Mayer et al. (2012) PloS One 7(1) available at www.plosone.org; Putnam et al. (2009) Diabetes 58:652-662; Walker et al. (2005) Proc. Nat'l. Acad. Sci. 102(11):4103-4108.

In particular aspects, either CD4⁺ and/or CD8⁺ precursor cells are isolated from the peripheral blood lymphocytes of an individual and are induced to become T-reg cells. In certain aspects, the individual is the patient. In other aspects, the individual is a healthy donor compatible with the patient. In still other aspects, the cells are isolated from a cell bank. In some aspects, the T-reg cells are expanded. In an preferred aspect, the T-reg cells are able to train other T-cells to become T-reg cells.

In some aspects, T-reg cells or precursor cells are separated from the peripheral blood before training and/or expansion in the presence of a compound identified by the methods described herein. T-reg cells or precursor cells can be isolated by any method known in the art. In some aspects, T-reg cells and/or precursor CD4⁺ and/or CD8⁺ cells are isolated and purified by any technique known in the art. Methods of characterizing phenotypes of isolated and purified cells will be known to the skilled artisan and include positive or negative selection with magnetic beads and/or flow cytometry. *See, e.g.,* Cao et al. (2010) Clinical Immunol. 136:329-337; Di Ianni et al. (2012) Transfusion and Apheresis Science 47:213-216; Walker et al. (2005) P.N.A.S. 102(11):4103-08; Chai et al. (2008) J. Immunol. 180:858-869; Tang et al. (2004) J. Exp. Med. 199(11):1455-65; Lin et al. (2003) Eur. J. Immunol. 33:626-638.

In certain aspects, T-reg cells or precursor cells of a particular phenotype are enriched by, for example, negative selection based on cell surface markers using AutoMACS technology (Miltenye Biotec) and/or FACs. In certain aspects, T-reg cells are enriched by negative selection (*e.g.*, by removing cells with markers that are not present on T-reg cells) followed by positive selection (*e.g*., by isolating cells using an antibody specific for a marker that is present on T-cells, such as CD25). In some aspects, T-reg cells are expanded by incubation with anti-CD3 and/or anti-CD28 antibodies, for example, antibodies coupled to paramagnetic beads, in the presence of IL-2 followed by FACs analysis of various cell markers (*e.g*., CD25 and/or CD4). In various aspects, the T-cells are expanded and/or trained and expanded in the presence of one or more enhancers. In some aspects, the enhancer is high molecular weight hyaluronic acid. As used herein, the term "high molecular weight hyaluronic acid" refers to hyaluronic acid having a molecular weight of at least about 1 × 10⁶ Da, such as of at least about 2 × 10⁶ Da, at least about 3 × 10⁶ Da, at least about 4 × 10⁶ Da, or more. *See e.g.,* Bollyky et al. (2007) J. Immunol. 179:744-747. Other enhancers include IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof. *See, e.g.,* Viney et al. (1998) J. Immunol. 160(12):5815-25; Horwitz et al. (2004) Seminars in Immunol. 16:135-143; Daniel et al. (2007) J. Immnol. 178(2): 458-68; Weiner et al. (2011) Immunol Rev. 241(1):241-259; Ma et al. (2011) Int. Immunopharmacol. 11(5):618-29; Adriouch et al. (2011) Front. Microbiol. 2:199; Dons et al. (2012) Human Immunol. 73:328-334 . In certain aspects, the enhancer is a sphingosine kinase 1 inhibitor as disclosed in U.S. Patent No. 8,872,888.

In certain aspects, the T-reg cells or precursor cells are isolated and expanded by at least about 10-fold, such as by at least about 20-fold, by at least about 30-fold, by at least about 40-fold, by at least about 50-fold, by at least about 60-fold, by at least about 70-fold, by at least about 80-fold, by at least about 90-fold or by at least about 100-fold before therapeutic administration.

T-reg cells can be expanded by any method known in the art. In a particular aspect, the cells are expanded *in vitro* in an isotonic medium such as CellGro medium, supplemented with autologous serum (10%) in the presence of IL-2 and clinical-grade anti CD3/anti-CD28 beads (1:1 ratio with cells). Cells are expanded for at least 7 days, for at least 8 days, for at least 9 days, for at least 10 days, for at least 11 days, for at least 12 days, for at least 13 days or for at least 14 days. In some aspects, cells are not expanded past 14 days. In some aspects, expanded cells are tested for their ability to suppress INF-y production and also for microbial contamination before infusion.

In various aspects, T-reg cells that are either expanded or not expanded are administered in an amount to achieve a T-reg/T-resp ratio in the blood of the patient of about 0.01, such as of about 0.05, of about 0.1, of about 0.25, of about 0.5, of about 0.75 or of about 1. The skilled artisan will understand that this ratio will depend on a number of factors, including but not limited to, the nature and severity of the autoimmune disease, the potency of the T-reg cells and the potency of the T-resp cells, and must be optimized for the particular individual and disease state.

In certain aspects, T-reg cells are infused at a dose of at least about 0.1 × 10⁵ /kg body weight, such as a dose of at least about 5 × 10⁵ cells/kg body weight, at least about 10 × 10⁵ cells/kg, at least about 20 × 10⁵ cells/kg body weight, at least about 30 × 10⁵ cells/kg body weight, at least about 40 × 10⁵ cells/kg body weight, at least about 50 × 10⁵ cells/kg body weight, at least about 60 × 10⁵ cells/kg body weight, at least about 70 × 10⁵ cells/kg body weight, at least about 80 × 105 cells/kg body weight, at least about 90 × 105 cells/kg body weight, at least about 10 × 10⁶ cells/kg body weight, at least about 15 × 10⁶ cells/kg body weight, or at least about 20 × 10⁶ cells/kg body weight or more.

T-reg cells are typically to be administered by injection or intravenous infusion. For infusion, T-reg cells are to be administered in a sterile, isotonic solution, for example, normal saline (*e.g.*, 0.9% NaCl) and 5% human albumin or lactated Ringer's solution.

In some aspects, inhibitory effects from an injection of trained and/or expanded T-reg cells can persist for at least about 1 week, such as for at least about 2 weeks, for at least about 3 weeks, for at least about 1 month or more. In particular aspects, the patient's blood is tested periodically to determine whether the expanded T-reg cells continue to exert inhibitory effects, and additional injections of T-reg cells are to be administered when needed.

In certain aspects, the T-reg cells are administered in conjunction with an additional therapeutically active agent. An additional therapeutically active agent for administration in conjunction with T-reg cells will depend on a number of factors known to the skilled artisan, including, but not limited to, the autoimmune disease being treated, the stage of the disease, and the overall health of the patient. Appropriate therapeutic agents will be known to the skilled practitioner. In some aspects, the additional agent is a dietary supplement such as a vitamin, a mineral, or an ω-3 fatty acid. In other aspects, the second therapeutically active agent is an anti-inflammatory agent, *e*.*g*., a corticosteroid. In particular aspects, the T-reg cells are to be administered with an enhancer.

### 5.5. Combination compound and cell-based therapy

In certain aspects, the patient is to be treated by administering (a) an effective amount of T-reg cells; and (b) an effective amount of a compound comprising an epitope that induces immune tolerance identified by the methods described herein. In certain aspects, the T-reg cells are not trained before administration. In various aspects, the T-reg cells are expanded, but are not trained, before administration. In still other aspects, the T-reg cells are trained before administration in the presence of a compound comprising an epitope that induces immune tolerance as identified by the methods described herein. In other aspects, the T-reg cells are trained and/or expanded *in vivo* in the presence of a compound as identified herein. Accordingly, in these aspects, a compound as identified herein is to be administered before, concurrently with or subsequent to administration of the T-reg cells. In some aspects, T-reg cells are trained *in vitro* and the compound used to train the T-reg cells for the combination therapy is identical to the compound that is to be administered to the patient. In other aspects, the compound used to train the T-reg cells for the combination therapy is different from the compound that is to be administered to the patient. In various aspects, the epitope of the compound used to train T-reg cells for combination therapy is identical to the epitope of the compound that is to be administered to the patient. In other aspects, the epitope of the compound used to train T-reg cells for combination therapy is different from the epitope of the compound that is to be administered to the patient.

In certain aspects, the T-reg cells are selected from the group consisting of CD4⁺CD25⁺ T-cells, CD4⁺Foxp3⁺ T-cells, CD4⁺CD25⁺Foxp3⁺ T-cells, IL-10 producing CD4⁺ Tr1 cells, TGF-β producing Th3 cells, CD8⁺ NKT cells, CD4⁻CD8⁻ T-cells, γδ T-cells, thymic nT-reg cells, periphery induced i-Treg cells, tolerogenic dendritic cells (DC), CD4⁺CD127^{lo/-} T-cells, CD4⁺CD127^{lo/-}CD25⁺ T-cells, CD45RA⁺ subset of CD4⁺CD127^{lo/-}CD25⁺ T-cells and mixtures thereof.

In some aspects, the T-reg cells and the compound are to be administered concurrently. In other aspects, the T-reg cells and the compound are to be administered consecutively. In certain aspects when the T-reg cells and the compound are administered consecutively, the compound is to be administered before the T-reg cells. In these aspects, the T-reg cells are to be administered at least about 1 hour, such as least about 1 day, at least about 1 week, or at least about 1 month or more after administration of the compound. In other aspects when the T-reg cells and the compound are to be administered consecutively, the T-reg cells are to be administered first and the compound is to be administered subsequent to the cell therapy. In various aspects, the compound is to be administered at least about 1 hour, such as least about 1 day or at least about 1 week, or at least about 1 month or more after administration of the compound. In these aspects, the duration of time between administration of the T-reg cells and the compound is informed by the population of T-reg cells in the patient's blood over time. Thus, as part of either a monotherapy or a combination therapy T-reg cells from the patient's blood can be isolated, counted and assayed for their ability to suppress T-resp cells.

In various aspects, administration of a compound to a patient who has received either cell therapy alone or a combination of compound and cell therapy is utilized to maintain a healthy number of active T-reg cells in the patient's peripheral blood over time, such as over about 2 weeks, or about 1 month, or about 2 months, or about 3 months or more. Thus, in these aspects, the compound is administered multiple times after the initial therapy, such as about once per week, twice per week, or every day for a period of time, *e*.*g*., 1 week, 1 month, 6 months or 1 year or more. It will be understood by a person of skill in the art that, in the combination therapy the T-reg cells and the compound can be administered in different formulations and by different routes, *e*.*g*., the T-reg cells are to be administered by infusion and the peptide is to be orally administered, or in the case of concurrent administration, the T-reg cells and the compound are to be administered in the same formulation, for example, by infusion.

In various aspects, the T-reg cells and/or the compound can be administered in the presence of an enhancer. In some aspects, the enhancer is high molecular weight hyaluronic acid. As used herein, the term "high molecular weight hyaluronic acid" refers to hyaluronic acid having a molecular weight of at least about 1 × 10⁶ Da, such as of at least about 2 × 10⁶ Da, at least about 3 × 10⁶ Da, at least about 4 × 10⁶ Da, or more. *See e.g.,* Bollyky et al. (2007) J. Immunol. 179:744-747. Other enhancers include
IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof. *See, e.g.,* Viney et al. (1998) J. Immunol. 160(12):5815-25; Horwitz et al. (2004) Seminars in Immunol. 16:135-143; Daniel et al. (2007) J. Immnol. 178(2): 458-68; Weiner et al. (2011) Immunol Rev. 241(1):241-259; Ma et al. (2011) Int. Immunopharmacol. 11(5):61 8-29; Adriouch et al. (2011) Front. Microbiol. 2:199; Dons et al. (2012) Human Immunol. 73:328-334 . In certain aspects, the enhancer is a sphingosine kinase 1 inhibitor as disclosed in U.S. Patent No. 8,872,888.

The compound can be administered as a single dose or as a divided dose as needed. In one aspect, an effective dosage is administered once per month. In another aspect, the effective dosage is to be administered once per week, or twice per week or three times per week. In another aspect, an effective dosage amount is to be administered about every 24 h. In another aspect, an effective dosage amount is to be administered about every 12 h. In certain aspects, more than one compound can be administered. Thus, the effective dosage amounts described herein refer to total amounts to be administered; that is, if more than one compound is to be administered, the effective dosage amounts correspond to the total amount to be administered. In certain aspects, the patient's blood is tested periodically to detect the presence and number of T-reg cells and the dosage and administration of a compound is to be administered based on the results.

It will be understood by the skilled artisan that, with combination therapies where compounds and T-reg cells are to be administered separately, the route, duration and frequency of dosing regimens may differ. For example, a compound may be orally administered once per day for 6 months, while T-reg cells may be administered by infusion once per month for a year.

### 5.6. Treatment of immune diseases of the eye

In particular aspects, the disclosure relates to uses in treating AMD in a patient by administering a compound identified by the methods described herein. In another aspect, the disclosure relates to uses in treating AMD in a patient wherein T-reg cells that have been expanded in the presence of a compound identified by the methods described herein are to be administered. In yet another aspect, the disclosure relates to uses in treating AMD in a patient by administering a compound and T-reg cells in a combination therapy, wherein the compound is identified by a method described herein, and wherein, in some aspects, the T-reg cells are trained in the presence of a compound identified by a method described herein. In other aspects, T-reg cells are expanded, but are not trained.

In another particular aspect, the disclosure relates to uses in treating uveitis in a patient by administering a compound identified by the methods described herein. In another aspect, the disclosure relates to uses in treating uveitis in a patient by administering T-reg cells that have been expanded in the presence of a compound identified by the methods described herein. In yet another aspects, the disclosure relates to uses in treating uveitis in a patient by administering a compound and T-reg cells in a combination therapy, wherein the compound is identified by a method described herein, and wherein, in some aspects, the T-reg cells are trained in the presence of a compound identified by a method described herein. In other aspects, T-reg cells are expanded, but are not trained.

In certain aspects, a compound that induces immune tolerance in a human patient suffering from AMD or uveitis is identified *in vitro* from a library or collection of compounds by identifying a compound that induces a response from a T-resp cell of the patient that is greater than a response from a T-resp cell of a healthy individual, wherein the compound that induces a response from a T-resp cell of the patient that is greater than the response from a T-resp cell of the healthy individual is identified as the compound that induces immune tolerance. In other aspects, a compound that induces immune tolerance in a human patient suffering from AMD or uveitis is identified *in vitro* from a library or collection of compounds by identifying a compound that induces a response from a T-reg cell of a healthy individual that is greater than a response from a T-reg cell of the patient, wherein the compound that induces a response from a T-reg cell of the healthy individual that is greater than the response from a T-reg cell of the patient is identified as the compound that induces immune tolerance in the patient.

In some aspects, the T-reg cells are autologous to the patient. In other aspects, the T-reg cells are heterologous and compatible to the patient and are from a healthy individual. In certain aspects, precursor cells are trained to become T-reg cells, as described above. In various aspects, the T-reg cells are antigen-specific. In other aspects, the T-reg cells are not antigen-specific.

In still other aspects, a patient suffering from AMD or uveitis can be treated by a combination of a compound as identified by the methods described herein and T-reg cells. In some aspects, the therapies are to be administered concurrently. In other aspects, the therapies are to be administered consecutively. In various aspects, the T-reg cells are trained and the epitope of the compound used to train T-reg cells for combination therapy is identical to the epitope of the compound that is to be administered to the patient in the combination therapy. In other aspects, the T-reg cells are trained and the epitope of the used to train T-reg cells for combination therapy is different from the epitope of the compound that is to be administered to the patient. In still other aspects of the monotherapy and the combination therapy, T-reg cells are not trained before administration. In certain aspects, T-reg cells are expanded but are not trained before administration. In some aspects, T-reg cells are trained *in vivo* by administering a compound as described herein before, concurrently with or subsequent to administration of untrained T-reg cells.

In other aspects when the T-reg cells and the compound are to be administered consecutively, the T-reg cells are administered first and the compound is to be administered subsequent to the cell therapy. In various aspects, the compound is to be administered at least about 1 hour, such as least about 1 day or at least about 1 week, or at least about 1 month or more after administration of the compound. In these aspects, the duration of time between administration of the T-reg cells and the compound are informed by the population of T-reg cells in the patient's blood over time. Thus, as part of the combination therapy T-reg cells from the patient's blood can be isolated, counted and assayed for their ability to suppress T-resp cells. Accordingly, in one aspect, administration of a compound to a patient who has received cell therapy alone or a combination of compound and cell therapy is utilized to maintain a healthy number of active T-reg cells in the patient's peripheral blood over time, such as about 2 weeks, or about 1 month, or about 2 months, or about 3 months or more.

In various aspects, the T-reg cells and/or the compound can be administered with an enhancer. In some aspects, the enhancer is high molecular weight hyaluronic acid. As used herein, the term "high molecular weight hyaluronic acid" refers to hyaluronic acid having a molecular weight of at least about 1 × 10⁶ Da, such as of at least about 2 × 10⁶ Da, at least about 3 × 10⁶ Da, at least about 4 × 10⁶ Da, or more. *See e.g.,* Bollyky et al. (2007) J. Immunol. 179:744-747. Other enhancers include IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof. *See, e.g.,* Viney et al. (1998) J. Immunol. 160(12):5815-25; Horwitz et al. (2004) Seminars in Immunol. 16:135-143; Daniel et al. (2007) J. Immnol. 178(2): 458-68; Weiner et al. (2011) Immunol Rev. 241(1):241-259; Ma et al. (2011) Int. Immunopharmacol. 11(5):618-29; Adriouch et al. (2011) Front. Microbiol. 2:199; Dons et al. (2012) Human Immunol. 73:328-334. In certain aspects, the enhancer is a sphingosine kinase 1 inhibitor as disclosed in U.S. Patent No. 8,872,888.

In particular aspects, the compound is a peptide. In specific embodiments as defined in the claims, the peptide is an S-antigen peptide. In other specific aspects, the peptide is an HLA-B27 peptide. In a particular embodiment as defined in the claims, the peptide is P-23. In another embodiment as defined in the claims, the peptide that is utilized in the expansion and/or conditioning of T-reg cells is P-23.

In certain aspects, the compound and/or cell therapy is to be administered with an additional therapeutically active agent. In some aspects, the second therapeutically active agent is a dietary supplement such as a vitamin, *e.g*., vitamin A, vitamin C, vitamin E, β-carotene or a mineral, *e.g*., zinc oxide or copper, or an ω-3 fatty acid. In other aspects, the second therapeutically active agent is an anti-VEGF drug. In still other aspects, the second therapeutically active agent is an anti-inflammatory drug, *e.g.,* a corticosteroid.

In certain aspects, the patient has early AMD, characterized by medium drusen (63-125 µm) without pigmentary abnormalities thought to be related to AMD. In other aspects, the patient has intermediate AMD, characterized by large drusen or with pigmentary abnormalities associated with at least medium drusen. In still other aspects, the patient has late AMD, characterized by lesions associated with neovascular AMD or geographic atrophy. Drusen, which are yellow or white accumulations of extracellular material that build up between Bruch's membrane and the retinal pigment epithelium of the eye, can be measured by any technique known by the skilled artisan. In certain aspects, drusen volumes are measured by spectral domain optical coherence tomography (SD-OCT). In other aspects, the patient has wet AMD.

In various aspects, treatment of AMD or uveitis refers to cessation of disease progression, for example, progression from early AMD to intermediate AMD or progression from intermediate AMD to late AMD or cessation of neovascularization in wet AMD.

### 5.7. Diagnosis, Prognosis, Monitoring and Kits

In certain aspects, the present disclosure relates to methods of diagn prognosticating or monitoring disease. In some aspects, a patient is diagnosed as having an autoimmune disease by a method comprising the steps of (a) measuring a response (RespH) from responder T-cells of a healthy individual and measuring a response (RespP) from responder T-cells of the patient; (b) measuring a response (RegH) from regulatory T-cells of a healthy individual and measuring a response (RegP) from regulatory T-cells of the patient; or (c) measuring a response (RespH) from responder T-cells of a healthy individual, a response (RespP) from responder T-cells of the patient and measuring a response (RegP) from regulatory T-cells of the patient and a response (RespH) from responder T-cells of a healthy individual in the presence of a compound comprising an epitope that induces immune tolerance in a human patient, wherein a comparison of RespH and RespP, or of RegH and RegP, or of both RespH and RespP and RegH and RegP that indicates a deviation of the patient's response from that of a healthy individual is indicative of an autoimmune disease in a patient. In other aspects, a patient is predicted to have an autoimmune disease by a method comprising the steps of (a) measuring a response (RespH) from responder T-cells of a healthy individual and measuring a response (RespP) from responder T-cells of the patient; (b) measuring a response (RegH) from regulatory T-cells of a healthy individual and measuring a response (RegP) from regulatory T-cells of the patient; or (c) measuring a response (RespH) from responder T-cells of a healthy individual, a response (RespP) from responder T-cells of the patient and measuring a response (RegP) from regulatory T-cells of the patient and a response (RespH) from responder T-cells of a healthy individual in the presence of a compound comprising an epitope that induces immune tolerance in a human patient, wherein a comparison of RespH and RespP, or of RegH and RegP, or of both RespH and RespP and RegH and RegP that indicates a deviation of the patient's response from that of a healthy individual is predictive of an autoimmune disease in a patient. In still other aspects, a patient suffering from an autoimmune disease is monitored, *e.g*., to determine the efficacy of a therapy and/or to determine disease progression by a method comprising the steps of (a) measuring a response (RespH) from responder T-cells of a healthy individual and measuring a response (RespP) from responder T-cells of the patient; (b) measuring a response (RegH) from regulatory T-cells of a healthy individual and measuring a response (RegP) from regulatory T-cells of the patient; or (c) measuring a response (RespH) from responder T-cells of a healthy individual, a response (RespP) from responder T-cells of the patient and measuring a response (RegP) from regulatory T-cells of the patient and a response (RespH) from responder T-cells of a healthy individual in the presence of a compound comprising an epitope that induces immune tolerance in a human patient, wherein a comparison of RespH and RespP, or of RegH and RegP, or of both RespH and RespP and RegH and RegP that indicates a deviation of the patient's response from that of a healthy individual is predictive of disease progression or efficacy of therapy.

The skilled artisan will understand that a RegP response that is greater than or equal to a RegH and/or a RespP that is lower than or equal to a RespH is indicative of the absence of an autoimmune disease in the patient, and/or no predicted autoimmune disease and/or diminishment of or lack of autoimmune disease progression in a patient. Conversely, a RegP response that is less than a RegH response and/or a RespP response that is greater than a RespH response is indicative of the presence of an autoimmune disease in the patient, and/or a predicted autoimmune disease and/or progression of an autoimmune disease in a patient.

In certain embodiments, the present disclosure relates to kits diagnosing, prognosticating or monitoring disease in a patient. In various embodiments, the kits described herein comprise one or more of: (a) a compound comprising an epitope that induces immune tolerance in a human patient; (b) a buffer; (c) a cell growth medium; (d) regulatory T-cells from an healthy individual; (e) responder T-cells from a healthy individual; and (f) an enhancer selected from the group consisting of high molecular weight hyaluronic acid, IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof.

### 6. EXAMPLES

This section will describe the various different working examples.
Examples which do not fall under the subject-matter of the appended claims do not form part of the present invention.

### 6.1. Example 1: Immune cell reactivity to antigens in AMD patients

In our evaluation of patients with AMD, we have noted marked immunological similarities to patients with uveitis. In AMD patients, we have seen activation of the acquired immune system, evidence of antigen sensitization as measured by proliferative responses by T cells, elevation of IL-17 cytokines and other members of that family, upregulation of IL-17RC in the macula, and an M2 to M1 macular switch, all of which are seen in uveitis. These characteristics make AMD a promising candidate for down-regulatory immune therapy with oral administration of antigen. Accordingly, peripheral blood lymphocytes of AMD patients were tested to determine whether they would manifest the same type of response that blood cells from uveitis patients did in an earlier study. *See* deSmet et al. (2001) Investigative Ophthalmology &Visual Science 42(13):3233-38.

### Materials and Methods

Several 18-mer sequences were constructed from the sequence of the retinal S-antigen. The fragments were chosen based on the responses of blood cells from uveitis patients previously tested. *See* deSmet *et al.* 2001. Whole blood was collected from patients with dry AMD and healthy controls. All AMD patients had either small drusen, intermediate drusen or large drusen.

Mononuclear lymphocytes were separated on isolymph gradient (Gallard-Schlesinger, Carle Place, NY) from heparinized blood shortly after the sample was obtained. Cells were resuspended in RMPI 1640 with HEPES (Gibco, Grand Island, NY), supplemented with glutamine (2 mM), Penicillin (100 U/ml), streptomycin (100 µg/ml), and 10% commercial heat-inactivated human AB serum (Biocell Laboratories, Carson, CA). These cells were immediately placed in culture at a density of 2 × 10⁵ cells/well in the presence of antigen, in flat-bottomed, 96-well plates (Costar, Cambridge, MA). All assays were plated in triplicate. Antigen concentrations were either 20 or 100 µg/ml. Peptides were tested simultaneously. For control of immune reactivity, purified protein derivative (PPD; Parke-Davis, Morris Plains, NJ) and purified phytohemagglutinin (PHA; Murex Diagnostics, Dartford, UK) were also tested. For the last 12 hours before harvesting at day 5, each well was pulsed with [3H]thymidine (2Ci/mmol, 0.5 µCi per 10 µl/well; New England Nuclear, Boston, MA).

### Results

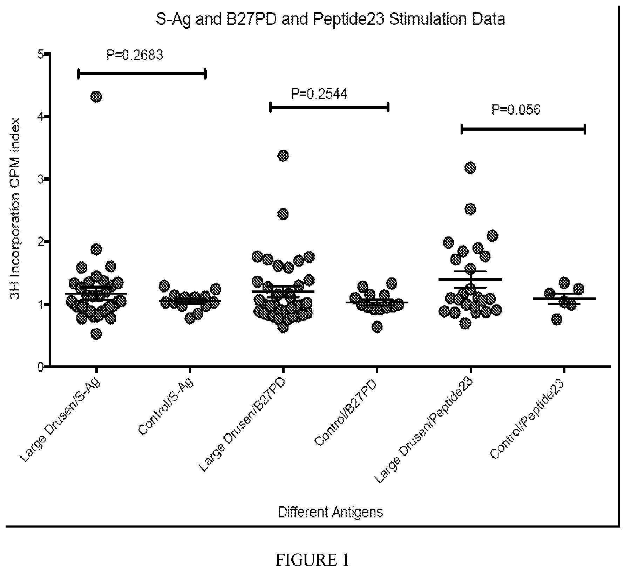

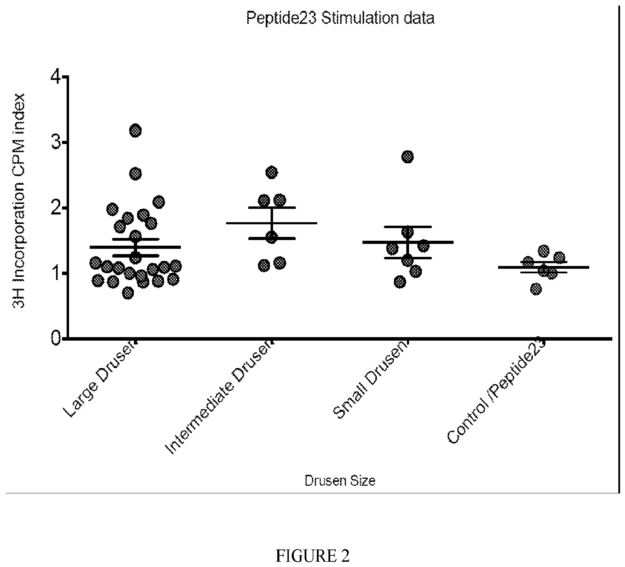

As shown in FIGS. 1 and 2, AMD patients' lymphocytes proliferated in the presence of whole S-antigen as well as to the BP27PD and Peptide 23 ("P-23") fragments. A large number of AMD patients responded best to P-23. In addition, FIG. 2 shows that lymphocytes from a significant number of AMD patients with small, intermediate and large drusen proliferated in the presence of P-23, and that lymphocytes from patients with large drusen had the best response.

### 6.2. Example 2: Peptide P-23 as AMD oral tolerizing agent

The objective of this study is to evaluate the safety and efficacy of the peptide P-23 as a long term method to prevent the development of more advanced AMD employing oral tolerance. Oral tolerance is investigated in patients with intermediate drusen who have a high risk of developing intermediate (large drusen with or without pigmentary changes) or late AMD. The primary outcome is the development of large drusen or late AMD. An important secondary outcome is defined as a mean change drusen volume on SD-OCT over 5 years without progression to geographic atrophy or neovascular disease. Participants who progress to advanced disease are considered treatment failures, and censured from the drusen change analysis at the time late AMD develops.

Drusen is measured by the use of the scanning laser ophthalmoscopy (SLO). In a pilot study, drusen number and area grades were significantly higher using the right side (AR) and left side (AL) in which the laterally scattered light is captured (retromode). *See* Diniz et al. (2013) Br. J. Ophthalmol. 97(3):285-90. Use of the lateral confocal aperture may highlight subclinical drusen and aid in monitoring disease progression and response to emerging non-neovascular AMD therapies.

### Target Population

Participants have early and intermediate AMD with intermediate drusen in both eyes or large drusen (with or without pigment changes) in one eye and intermediate drusen in the fellow eye. All study eyes have intermediate drusen (<63 µm).

### Methods

This is a 5-year double-masked randomized clinical trial of 145 participants to assess the safety and efficacy of oral tolerance induction using drusen volume on OCT as a clinical end point. Patients are randomized 1:1 to P23 fragment of retinal S-Antigcn 4 mg oral daily or placebo oral daily.

### Study Outcome

The primary outcome is the development of large drusen or late AMD in patients with bilateral medium drusen or eyes whose fellow eye has large drusen. An important secondary outcome is defined as a mean change drusen volume on SD-OCT over 5 years without progression to geographic atrophy or neovascular disease. Participants who progress to advanced disease are considered treatment failures, and censured from the drusen change analysis at the time late AMD develops.

Other secondary outcomes in study eyes include:
- Progression from intermediate drusen to large drusen or late AMD
- Change in Dark Adaptation time
- Mean change in best-corrected ETDRS (Early Treatment of Diabetic Retinopathy Study protocol) visual acuity from baseline to year-1.
- Changes in autofluorescence patterns on fundus autofluorescence photography
- Correlation with levels of serum inflammatory cytokines
- Correlation with flow cytometry evaluating T regulatory cells
- Correlation with epigenetic changes (demethylation of interleukin-17 receptor C)
- Changes in chromaticity coordinates on Cambridge color test (Regan et al., 1994)
- Safety outcomes
- Changes in drusen volume through year-5 (USC protocol)

### Sample Size Consideration

Detecting a 50% decrease in the development of large drusen or late AMD in patients with bilateral medium drusen or eyes whose fellow eye has large drusen, requires 132 patients with an α of 0.05 and β of 0.2. A 10% adjustment for loss to follow-up and non-compliance would increases the required sample size to 145.

Using a 0.041 mm change in cube root drusen volume, as compared to the reference mean change, has a power of 80.6%. This is based on a 0.16 mm mean change in cube root volume for this drusen size population (Yehoshua and Gregori, 2011) and reflects a mean change of -0.025 mm in cube root volume in the treatment group.

### Hazards and Discomforts

Possible complications associated with the study may include:
- Temporary gastrointestinal upset from either placebo and/or P23.
- Transient ocular discomfort from ocular examination.
- Temporary discomfort, bruising or infection from blood draw.

### 6.3. Example 3: Activation of T-reg cells and analysis of suppressive function

### Preparation of cells and solutions

CD4⁺CD25⁻ and CD4⁺CD25⁺ T-cell suspensions in RPMI-10 are prepared as described in Thornton (2003) Current Protocols in Immunology, Unit 3.5A (DOI: 10.1002/0471 142735.im0305as57). Cells are counted and CD4⁺CD25⁻ and CD4⁺CD25⁺ cells are adjusted to 1 × 10⁶ cells/mL with RPMI-10 medium.

Accessory cells in RPMI-10 are prepared as described in Thornton (2003). Cells are counted and adjusted to 1 × 10⁶ cells/mL with RPM1-10. The following working solutions are prepared: 1 µg/mL anti-CD3 in RPMI-10; 200 U/mL IL-2 in RPMI-10; and 2 µg/mL anti-CD28 in RPMI-10.

50 µL of CD4⁺CD25⁻ cells are added to each of nine wells of a 96-well flat-bottom microtiter plate and 50 µL of CD4⁺ CD25⁺ cells are added to each of nine wells of a 96-well flat-bottom microtiter plate. 50 µL of accessory cells and 50 µL of 1 50 µg/mL anti-CD3 are added to each of the wells. 50 µL of 200 U/mL IL-2 are added to three wells of the CD25⁻cells and three wells of the CD25⁺ cells.

### Suppressive function assay

50 µL of CD4⁺CD25⁺ cells are added to three wells of a 96-well microtiter plate. A series of 3-4 two-fold dilutions of the CD4⁺CD25⁺ cells are made and control wells containing only 50 µL of RPMI-10 medium are included. After serial dilution, the starting number of cells in the wells are: 5 × 10⁴ CD25⁺ cells/well, 2.5 × 10⁴ CD25⁺ cells/well, 1.25 × 10⁴ CD25⁺ cells/well, 0.625 × 10⁴ CD25⁺ cells/well and 0.3 × 10⁴ CD25⁺ cells/well.

50 µL of CD4⁺CD25⁻ cells, 50 µL of accessory cells and 50 µL of 1 µg/mL anti-CD3 are added to the wells. The microtiter plates are placed in a 37°C, 5%-7% CO₂ humidified incubator for 3 days (about 66 hours).

On the morning of the third day [³H]thymidine is added to each well and plates are returned to the incubator to pulse for 6-8 hours. Cells are harvested using a semi automated sample harvester and counts per minute are measured in a β scintillation counter.

### Results

CD4⁺CD25⁺ cells are non-responsive to stimulation with anti-CD3 and accessory cells. Addition of anti-CD28 to CD4⁺CD25⁺ cells stimulated with anti-CD3 and accessory cells does not restore proliferation of these cells. The addition of anti-CD28 to CD4+CD25-cells enhances their proliferation. Addition of IL-2 to CD4⁺CD25⁺ cells results in proliferation of these cells. Addition of CD4⁺CD25⁺ cells to CD4⁺CD25⁻ cells results in a dose-dependent decrease in the proliferation of CD4⁺CD25⁻ cells.

### 6.4. Example 4: Activation and expansion of CD4+CD25+ T-cells and analysis of suppressive function

### Activation of CD4⁺CD25⁺ T-cells

CD4⁺CD25⁺ T-cells are purified in complete RMPI-10 medium supplemented with 100 U/mL IL-2 as described in Thornton (2003) Current Protocols in Immunology, Unit 3.5A (DOI: 10.1002/0471142735.im0305as57). CD4⁺CD25⁺ cells are counted and adjusted to 1 × 10⁶ cells/mL with RPM1-10/IL-2.

A working solution of 5 µg/mL anti-CD3 in PBS is prepared. 300 µL of anti-CD3 solution is added to each well of a 24-well plate. Number of wells to be coated is based on anticipated yield of CD4⁺CD25⁺ cells. Plates are incubated for 90 min in a 37°C, 5%-7% CO₂ humidified incubator. Antibody is removed from the plates and wells are washed 2x with PBS to remove excess antibody. 1 mL containing 1 × 10⁶ CD4⁺CD25⁺ cells are added to the wells. Plates are placed in a 37°C, 5%-7% CO₂ humidified incubator for 3 days. CD4⁺CD25⁺ cells are fully activated but are not greatly expanded.

After three days, cells are split 1:3 or 1:4 in RPMI-10 medium supplemented with 100 U/mL IL-2 and are returned to the a 37°C, 5%-7% CO₂ humidified incubator.

### Suppressive function assay

Activated CD4⁺CD25⁺ cells are harvested by pipetting up and down rigorously. Cells are centrifuged for 10 min at 200xg (Sorvall H-1000B rotor at approx. 1000 rpm) at 4°C. Cells are washed 2x to completely remove remaining IL-2 and resuspend in RPMI-10. Cells are adjusted to 1 × 10⁶ cells/mL with RPMI-10.

CD4⁺ T-cell suspension in RPMI-10 is prepared from TCR transgenic mice as described in Unit 3.5A of Thornton (2003). CD4⁺ cells are counted and adjusted to 1 × 10⁶ cells/mL with RPMI-10. Antigen at 4x is diluted to the desired final concentration with RPMI-10. 50µL of CD4⁺CD25⁺ cells are added to three wells of a 96-well microtiter plate. A series of 3-4 two-fold dilutions of CD4⁺CD25⁺ cells are made and control wells containing 50 µL of RPMI-10 are included. After serial dilution, the starting number of cells in the wells are: 5 × 10⁴ CD25⁺ cells/well, 2.5 × 10⁴ CD25⁺ cells/well, 1.25 × 10⁴ CD25⁺ cells/well, 0.625 × 10⁴ CD25⁺ cells/well and 0.3 × 10⁴ CD25⁺ cells/well.

50 µL TCR Tg CD4+ cells, 50 µL of accessory cells and 50 µL of antigen are added to each well. The microtiter plates are placed in a 37°C, 5%-7% CO₂ humidified incubator for 3 days (about 66 hours).

On the morning of the third day [³H]thymidine is added to each well and plates are returned to the incubator to pulse for 6-8 hours. Cells are harvested using a semiautomated sample harvester and counts per minute are measured in a β scintillation counter.

### 6.5. Example 5: In vitro identification of a compound comprising an epitope that induces immune tolerance

Overlapping oligomeric peptide determinants of human HLA-B27 (Accession no. CAA27578.1) spanning the length of the protein are synthesized on an automated peptide synthesizer (Intavis, AG, Koeln, Germany). Each peptide is 15 amino acids in length and overlaps the previous peptide by 3 amino acids. Peptides are purified by HPLC to at least 95% purity. The amino acid composition of peptides is verified using amino acid analysis and automated gas-phase sequencing.

CD4⁺CD25⁺ cells are prepared as described in Example 3, above. 50 µL of CD4⁺CD25⁺ cells, 50 µL of accessory cells and 50 µL of 1 µg/mL anti-CD3 are added to the wells of a 96-well microtiter plate. HLA-B27 peptide is added to each well except for the control wells. All peptides are assayed in triplicate (3 wells each). The microtiter plates are placed in a 37°C, 5%-7% CO₂ humidified incubator for 3 days (about 66 hours).

On the morning of the third day [³H]thymidine is added to each well and plates are returned to the incubator to pulse for 6-8 hours. Cells are harvested using a semiautomated sample harvester and counts per minute are measured in a β scintillation counter.

The peptide that elicits the largest CD4⁺CD25⁺ cell proliferation as measured by levels of [³H]thymidine as compared CD4⁺CD25⁺ cell proliferation in the absence of peptide is the peptide that elicits immune tolerance in a patient.

### 6.6. Example 6: Administration of T-reg cells to patients suffering from type-I diabetes mellitus

T-reg cells from partially HLA-matched healthy individuals are prepared as set forth in Trzonkowski et al. (2009) Clin. Immunol. 133:22-26 and Marek et a/. (2011) Cell Transplant 12:1747-1758. T-regs are cultured in the presence of 10% autologous serum, IL-2 (1000 U/mL) and clinical-grade anti-CD3/anti-CD28 beads in a 1:1 ratio with cells. Cells are cultured for about 10 days to 2 weeks, but no longer than 2 weeks.

T-reg cells for infusion are washed out completely, suspended in 250 mL 0.9% NaCl and transferred in slow infusion to a patient under anesthesia within 1 hour. T-regs are administered in a dose from 10 × 10⁶/kg body weight to 20 × 10⁶ /kg body weight.

The endpoint is fasting C-peptide, HbA_{1c} level and insulin requirement. The percentage of T-regs in the patient's blood after 2 weeks, 2 months, 4 months and 6 months is assayed. If the percentage of T-regs drops by 50%, a compound as identified by the methods described herein is to be administered to the patient.

## Claims

1. A pharmaceutical composition comprising the compound having SEQ ID NO: 1 for use in treating or preventing an autoimmune disease.

2. A pharmaceutical composition comprising an effective amount of regulatory T-cells and the compound having SEQ ID NO: 1 for use in treating or preventing an autoimmune disease.

3. The pharmaceutical composition for use of claim 2, wherein the regulatory T-cells are expanded.

4. The pharmaceutical composition for use of claim 2, wherein the regulatory T-cells are autologous to the patient.

5. The pharmaceutical composition for use of claim 2, wherein the regulatory T-cells are heterologous to and compatible with the patient.

6. The pharmaceutical composition for use of claim 1 or 2, wherein the autoimmune disease is selected from the group consisting of acute disseminated encephalomyelitis, Addison's disease, agammaglobulinemia, age-related macular degeneration, alopecia areata, amyotrophic lateral sclerosis, ankylosing spondylitis, antiphospholipid syndrome, anti synthetase syndrome, atopic allergy, atopic dermatitis, autoimmune aplastic anemia, autoimmune cardiomyopathy, autoimmune enteropathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune uticaria, autoimmune uveitis, Balo disease/Balo concentric sclerosis, Behcet's disease, Berger's disease, Bickerstaff s encephalitis, Blau syndrome, Bullous pemphigoid, cancer, Castleman's disease, celiac disease, Chagas disease, chronic inflammatory demyelinating polyneuropathy, chronic recurrent multifocal osteomyelitis, chronic obstructive pulmonary disease, Churg-Strauss syndrome, cicatricial pemphigoid, Cogan syndrome, cold agglutinin disease, complement component 2 deficiency, contact dermatitis, cranial arteritis, CREST syndrome, Crohn's disease, Cushing's syndrome, cutaneous leukocytoclastic angiitis, Dego's disease, Dercum's disease, dermatitis herpetiformis, dermatomyositis, diabetes mellitus type 1, diffuse cutaneous systemic sclerosis, Dressler's syndrome, drug-induced lupus, discoid lupus erythematosus, eczema, endometriosis, enthesitis-related arthritis, eosinophilic fasciitis, eosinophilic gastroenteritis, epidermolysis bullosa acquisita, erythema nodosum, erythroblastosis fetalis, essential mixed cryoglobulinemia, Evan's syndrome, fibrodysplasia ossificans progressive, fibrosing alveolitis, gastritis, gastrointestinal pemphigoid, glomerulonephritis, Goodpasture's syndrome, Graves' disease, Guillan-Barré syndrome, Hashimoto's encephalopathy, Hashimoto's thyroiditis, Henoch-Schonlein purpura, gestational pemphigoid, hidradenitis suppurativa, Hughes-Stovin syndrome, hypogammaglobulinemia, idiopathic inflammatory demyelinating diseases, idiopathic pulmonary fibrosis, idiopathic thrombocytopenic purpura, IgA nephropathy, inclusion body myositis, chronic inflammatory demyelinating polyneuropathy, interstitial cystitis, juvenile idiopathic arthritis, Kawasaki's disease, Lambert-Eaton myasthenic syndrome, leukocytoclastic vasculitis, lichen planus, lichen sclerosus, linear IgA disease, lupus erythematosus, Majeed syndrome, Menière's disease, microscopic polyangiitis, mixed connective tissue disease, morphea, Mucha-Habermann disease, multiple sclerosis, myasthenia gravis, myositis, narcolepsy, neuromyelitis optica, neuromyotonia, occular cicatricial pemphigoid, opsoclonus myoclonus syndrome, Ord's thyroiditis, palindromic rheumatism, pediatric autoimmune neuropsychiatric disorders associated with streptococcus, paraneoplastic cerebellar degeneration, paroxysmal nocturnal hemoglobinuria, Parry Romberg syndrome, Parsonage-Turner syndrome, Pars planitis, pemphigus vulgaris, pernicious anaemia, perivenous encephalomyelitis, POEMS syndrome, polyarteritis nodosa, polymyalgia rheumatic, polymyositis, primary biliary cirrhosis, primary sclerosing cholangitis, progressive inflammatory neuropathy, psoriasis, psoriatic arthritis, pyoderma gangrenosum, pure red cell aplasia, Rasmussen's encephalitis, Raynaud phenomenon, relapsing polychondritis, Reiter's syndrome, restless leg syndrome, retroperitoneal fibrosis, rheumatoid arthritis, rheumatic fever, sarcoidosis, schizophrenia, Schmidt syndrome, Schnitzler syndrome, scleritis, scleroderma, serum sickness, Sjogren's syndrome, spondyloarthropathy, stiff person syndrome, subacute bacterial endocarditis, Susac's syndrome, Sweet's syndrome, sympathetic ophthalmia, Takayasu's arteritis, temporal arteritis, thrombocytopenia, Tolosa-Hunt syndrome, transverse myelitis, ulcerative colitis, undifferentiated connective tissue disease, urticarial vasculitis, vasculitis, vitiligo and Wegener's granulomatosis.

7. The pharmaceutical composition for use of claim 1 or 2, wherein the autoimmune disease is age-related macular degeneration.

8. The pharmaceutical composition for use of claim 1 or 2, wherein the autoimmune disease is autoimmune uveitis.

## Patentansprüche

1. Arzneimittel, das die Verbindung mit SEQ ID NO:1 umfasst, zur Verwendung bei der Behandlung oder Vorbeugung einer Autoimmunerkrankung.

2. Arzneimittel, das eine wirksame Menge von regulatorischen T-Zellen und die Verbindung mit SEQ ID NO:1 umfasst, zur Verwendung bei der Behandlung oder Vorbeugung einer Autoimmunerkrankung.

3. Arzneimittel zur Verwendung nach Anspruch 2, wobei die regulatorischen T-Zellen expandiert sind.

4. Arzneimittel zur Verwendung nach Anspruch 2, wobei die regulatorischen T-Zellen dem Patienten autolog sind.

5. Arzneimittel zur Verwendung nach Anspruch 2, wobei die regulatorischen T-Zellen mit dem Patienten heterolog und kompatibel sind.

6. Arzneimittel zur Verwendung nach Anspruch 1 oder 2, wobei die Autoimmunerkrankung ausgewählt ist aus der Gruppe bestehend aus akuter disseminierter Enzephalomyelitis, Morbus Addison, Agammaglobulinämie, altersbedingter Makuladegeneration, Alopecia areata, amyotropher Lateralsklerose, Spondylitis ankylosans, Antiphospholipid-Syndrom, Antisynthetase-Syndrom, atopischer Allergie, atopischer Dermatitis, Autoimmun-Aplastische Anämie, Autoimmun-Kardiomyopathie, Autoimmunenteropathie, autoimmunhämolytische Anämie, Autoimmunhepatitis, Autoimmunerkrankung des Innenohrs, autoimmunem lymphoproliferativem Syndrom, autoimmuner peripherer Neuropathie, autoimmuner Pankreatitis, polyendokriner Autoimmunerkrankung, autoimmuner Progesteron-Dermatitis, autoimmunthrombozytopenischer Purpura, Autoimmunuticaria, Autoimmunuveitis, Balo-Krankheit/Balo-konzentrischer Sklerose, Behcet-Krankheit, Berger-Syndrom, Bickerstaff's Encephalitis, Blau-Syndrom, bullösem Pemphigoid, Krebs, Castleman-Krankheit, Zöliakie, Chagas-Krankheit, chronischer entzündlicher demyelinierender Polyneuropathie, chronisch wiederkehrender multifokaler Osteomyelitis, chronisch obstruktiver Lungenerkrankung, Churg-Strauss-Syndrom, vernarbendem Pemphigoid, Cogan-Syndrom, kalter Agglutinin-Krankheit, Komplementkomponente 2-Mangel, Kontaktdermatitis, cranialer Arteritis, CREST-Syndrom, Crohn-Krankheit, Cushing-Syndrom, kutaner leukozytoclastischer Angiitis, Dego-Krankheit, Dercum-Krankheit, Dermatitis herpetiformis, Dermatomyositis, Diabetes mellitus Typ 1, diffuser kutaner systemischer Sklerose, Dressler-Syndrom, drogeninduziertem Lupus, scheibenförmigem Lupus erythematodes, Ekzemen, Endometriose, Enthesitis-assoziierter Arthritis, eosinophiler Fasciitis, eosinophiler Gastroenteritis, Epidermolyse bullosa acquisita, Erythema nodosum, Erythroblastosis fetalis, essentieller gemischter Cryoglobulinämie, Evan-Syndrom, progressiver Fibrodysplasia ossificans, fibrosierender Alveolitis, Gastritis, gastrointestinalem Pemphigoid, Glomerulonephritis, Goodpasture-Syndrom, Graves-Krankheit, Guillan-Barré-Syndrom, Hashimoto-Enzephalopathie, Hashimoto-Thyreoiditis, Henoch-Schönlein Purpura, Pemphigoid gestationis, Hidradenitis suppurativa, Hughes-Stovin-Syndrom, Hypogammaglobulinämie, idiopathischen entzündlichen demyelinierenden Erkrankungen, idiopathischer Lungenfibrose, idiopathischer thrombozytopenischer Purpura, IgA-Nephropathie, Einschlusskörpermyositis, chronisch entzündlicher demyelinisierender Polyneuropathie, interstitieller Blasenentzündung, juveniler idiopathischer Arthritis, Kawasaki-Krankheit, Lambert-Eaton-Myasthen-Syndrom, leukozytoklastischer Vaskulitis, Lichen planus, Lichen sclerosus, linearer IgA-Krankheit, Lupus erythematodes, Majeed-Syndrom, Menière Krankheit, mikroskopischer Polyangiitis, gemischter Bindegewebserkrankung, Morphea, Mucha-Habermann-Krankheit, Multipler Sklerose, Myasthenia gravis, Myositis, Narkolepsie, Neuromyelitis optica, Neuromyotonie, okulärem vernarbendem Pemphigoid, Opsoclonus myoclonus-Syndrom, Ord-Thyreoiditis, palindromischem Rheuma, pädiatrischen autoimmunen neuropsychiatrischen Störungen im Zusammenhang mit Streptokokken, paraneoplastischer Kleinhirn-Degeneration, paroxysmaler nächtlicher Hämoglobinurie, Parry Romberg-Syndrom, Parsonage-Turner-Syndrom, Pars planitis, Pemphigus vulgaris, perniziöser Anämie, perivenöser Enzephalomyelitis, POEMS-Syndrom, Polyarteritis nodosa, rheumatischer Polymyalgie, Polymyositis, primärer biliärer Zirrhose, primärer sklerosierender Cholangitis, fortschreitender entzündlicher Neuropathie, Psoriasis, Psoriasis-Arthritis, Pyoderma gangrenosum, reiner Aplasie der roten Blutkörperchen, Rasmussen-Enzephalitis, Raynaud-Phänomen, rezidivierender Polychondritis, Reiter-Syndrom, Restless-Leg-Syndrom, retroperitonealer Fibrose, rheumatoider Arthritis, rheumatischem Fieber, Sarkoidose, Schizophrenie, Schmidt-Syndrom, Schnitzler-Syndrom, Skleritis, Sklerodermie, Serumkrankheit, Sjögren-Syndrom, Spondyloarthropathie, Stiff-Person-Syndrom, subakuter bakterieller Endokarditis, Susac-Syndrom, Sweet-Syndrom, sympathischer Ophthalmie, Takayasu-Arteritis, temporale Arteritis, Thrombozytopenie, Tolosa-Hunt-Syndrom, transversaler Myelitis, Colitis ulcerosa, undifferenzierter Bindegewebserkrankung, Urtikaria-Vaskulitis, Vaskulitis, Vitiligo und Wegener-Granulomatose.

7. Arzneimittel zur Verwendung nach Anspruch 1 oder 2, wobei die Autoimmunkrankheit altersbedingte Makulardegeneration ist.

8. Arzneimittel zur Verwendung nach Anspruch 1 oder 2, wobei die Autoimmunkrankheit autoimmune Uveitis ist.

## Revendications

1. Composition pharmaceutique comprenant le composé ayant la SEQ ID NO: 1 pour une utilisation dans le traitement ou la prévention d'une maladie auto-immune.

2. Composition pharmaceutique comprenant une quantité efficace de lymphocytes T régulateurs et le composé ayant la SEQ ID NO: 1 pour une utilisation dans le traitement ou la prévention d'une maladie auto-immune.

3. Composition pharmaceutique pour une utilisation selon la revendication 2, dans laquelle les lymphocytes T régulateurs sont expansés.

4. Composition pharmaceutique pour une utilisation selon la revendication 2, dans laquelle les lymphocytes T régulateurs sont autologues au patient.

5. Composition pharmaceutique pour une utilisation selon la revendication 2, dans laquelle les lymphocytes T régulateurs sont hétérologues au patient et compatibles avec celui-ci.

6. Composition pharmaceutique pour une utilisation selon la revendication 1 ou 2, dans laquelle la maladie auto-immune est choisie dans le groupe consistant en l'encéphalomyélite aiguë disséminée, la maladie d'Addison, l'agammaglobulinémie, la dégénérescence maculaire liée à l'âge, l'alopecia aerata, la sclérose latérale amyotrophique, la spondylarthrite ankylosante, le syndrome des antiphospholipides, le syndrome des antisynthétases, l'allergie atopique, la dermatite atopique, l'anémie aplasique auto-immune, la cardiomyopathie auto-immune, l'entéropathie auto-immune, l'anémie hémolytique auto-immune, l'hépatite auto-immune, la maladie auto-immune de l'oreille interne, le syndrome lymphoprolifératif auto-immun, la neuropathie périphérique auto-immune, la pancréatite auto-immune, le syndrome polyendocrinien auto-immun, la dermatose auto-immune à la progestérone, le purpura thrombopénique auto-immun, l'urticaire d'origine auto-immune, l'uvéite auto-immune, la maladie de Balo/la sclérose concentrique de Balo, la maladie de Behçet, la maladie de Berger, l'encéphalite de Bickerstaff, le syndrome de Blau, la pemphigoïde bulleuse, le cancer, la maladie de Castleman, la maladie coeliaque, la maladie de Chagas, la polyneuropathie inflammatoire démyélinisante chronique, l'ostéomyélite multifocale chronique récurrente, la bronchopneumopathie chronique obstructive, le syndrome de Churg-Strauss, la pemphigoïde cicatricielle, le syndrome de Cogan, la maladie des agglutinines froides, le déficit en composant du complément 2, la dermatite de contact, l'artérite crânienne, le syndrome de CREST, la maladie de Crohn, le syndrome de Cushing, l'angéite cutanée leucocytoclasique, la maladie de Dego, la maladie de Dercum, la dermatite herpétiforme, la dermatomyosite, le diabète sucré de type 1, la sclérodermie systémique cutanée diffuse, le syndrome de Dressler, le lupus induit par un médicament, le lupus érythémateux discoïde, l'eczéma, l'endométriose, l'arthrite liée à l'enthésite, la fasciite à éosinophiles, la gastro-entérite à éosinophiles, l'épidermolyse bulleuse acquise, l'érythème noueux, l'érythroblastose foetale, la cryoglobulinémie mixte essentielle, le syndrome d'Evan, la fibrodysplasie ossifiante progressive, l'alvéolite fibrosante, la gastrite, la pemphigoïde gastro-intestinale, la glomérulonéphrite, le syndrome de Goodpasture, la maladie de Grave, le syndrome de Guillain-Barré, l'encéphalopathie d'Hashimoto, la thyroïdite d'Hashimoto, le purpura d'Henoch-Schonlein, la pemphigoïde gestationnelle, l'hidradénite suppurée, le syndrome de Hughes-Stovin, l'hypogammaglobulinémie, les maladies inflammatoires démyélinisantes idiopathiques, la fibrose pulmonaire idiopathique, le purpura thrombopénique idiopathique, la néphropathie à IgA, la myosite à corps d'inclusion, la polyneuropathie inflammatoire démyélinisante chronique, la cystite interstitielle, l'arthrite juvénile idiopathique, la maladie de Kawasaki, le syndrome myasthénique de Lambert-Eaton, la vascularite leucocytoclasique, le lichen plan, le lichen scléreux, la maladie à IgA linéaires, le lupus érythémateux, le syndrome de Majeed, la maladie de Ménière, la polyangéite microscopique, la connectivité mixte, la maladie de morphée, la maladie de Mucha-Habermann, la sclérose en plaques, la myasthenia gravis, la myosite, la narcolepsie, la neuromyélite optique, la neuromyotonie, la pemphigoïde cicatricielle oculaire, le syndrome d'opsoclonie-myoclonie, la thyroïdite d'Ord, le rhumatisme palindromique, les troubles neuropsychiatriques auto-immuns pédiatriques associés au streptocoque, la dégénérescence cérébelleuse paranéoplasique, l'hémoglobinurie paroxystique nocturne, le syndrome de Parry Romberg, le syndrome de Parsonage-Turner, le Pars planitis, le pemphigus vulgaire, l'anémie pernicieuse, l'encéphalite périveineuse, le syndrome de POEMS, la polyartérite noueuse, la polymyalgie rhumatismale, la polymyosite, la cirrhose biliaire primaire, la cholangite sclérosante primaire, la neuropathie inflammatoire progressive, le psoriasis, l'arthrite psoriasique, le pyoderma gangrenosum, l'aplasie pure des globules rouges, l'encéphalite de Rasmussen, le phénomène de Raynaud, la polychondrite récidivante, le syndrome de Reiter, le syndrome de la jambe sans repos, la fibrose rétropéritonéale, la polyarthrite rhumatoïde, le rhumatisme articulaire aigu, la sarcoïdose, la schizophrénie, le syndrome de Schmidt, le syndrome de Schnitzler, la sclérite, la sclérodermie, la maladie sérique, le syndrome de Sjögren, la spondylarthropathie, le syndrome de la personne raide, l'endocardite bactérienne subaiguë, le syndrome de Susac, le syndrome de Sweet, l'ophtalmie sympathique, l'artérite de Takayasu, l'artérite temporale, la thrombocytopénie, le syndrome de Tolosa-Hunt, la myélite transverse, la colite ulcérative, la connectivité indifférenciée, la vascularite urticarienne, le vitiligo et la granulomatose de Wegener.

7. Composition pharmaceutique pour une utilisation selon la revendication 1 ou 2, dans laquelle la maladie auto-immune est la dégénérescence maculaire liée à l'âge.

8. Composition pharmaceutique pour une utilisation selon la revendication 1 ou 2, dans laquelle la maladie auto-immune est l'uvéite auto-immune.
